# EUROPÄISCHE PATENTANMELDUNG

(11) **EP 1 659 174 A2**
(43) Veröffentlichungstag der Anmeldung: **24.05.2006**
(21) Anmeldenummer: 05025017.4
(22) Anmeldetag: 16.11.2005
(51) Int. Cl.: C12N 9/10, C12P 13/12, C12R 1/15

(54) **Allele des metK-Gens aus coryneformen Bakterien**

(30) Priorität: 17.11.2004 DE 102004055414
(71) Anmelder: Degussa AG, 40474 Düsseldorf (DE)
(72) Erfinder: Schischka, Natalie, 33649 Bielefeld (DE); Bathe, Brigitte, Dr., 33154 Salzkotten (DE)

(57) **Zusammenfassung**

Die Erfindung betrifft Allele des metK-Gens aus coryneformen Bakterien kodierend für S-Adenosylmethionin-Synthetasen und Verfahren zur fermentativen Herstellung von L-Methionin unter Verwendung von Bakterien, die diese Allele enthalten.

SEQUENCE LISTING

<110>  Degussa AG

<120>  Alleles of the metK gene of coryneform bacteria

<130>  040313BT

<160>  18

<170>  PatentIn version 3.2

<210>  1
<211>  1224
<212>  DNA
<213>  Corynebacterium glutamicum


<220>
<221>  CDS
<222>  (1)..(1221)
<223>  metK wildtype gene

<400>  1
gtg gct cag cca acc gcc gtc cgt ttg ttc acc agt gaa tct gta act      48
Met Ala Gln Pro Thr Ala Val Arg Leu Phe Thr Ser Glu Ser Val Thr
1               5                   10                  15

gag gga cat cca gac aaa ata tgt gat gct att tcc gat acc att ttg      96
Glu Gly His Pro Asp Lys Ile Cys Asp Ala Ile Ser Asp Thr Ile Leu
            20                  25                  30

gac gcg ctg ctc gaa aaa gat ccg cag tcg cgc gtc gca gtg gaa act      144
Asp Ala Leu Leu Glu Lys Asp Pro Gln Ser Arg Val Ala Val Glu Thr
        35                  40                  45

gtg gtc acc acc gga atc gtc cat gtt gtt ggc gag gtc cgt acc agc      192
Val Val Thr Thr Gly Ile Val His Val Val Gly Glu Val Arg Thr Ser
    50                  55                  60

gct tac gta gag atc cct caa tta gtc cgc aac aag ctc atc gaa atc      240
Ala Tyr Val Glu Ile Pro Gln Leu Val Arg Asn Lys Leu Ile Glu Ile
65                  70                  75                  80

gga ttc aac tcc tct gag gtt gga ttc gac gga cgc acc tgt ggc gtc      288
Gly Phe Asn Ser Ser Glu Val Gly Phe Asp Gly Arg Thr Cys Gly Val
                85                  90                  95

tca gta tcc atc ggt gag cag tcc cag gaa atc gct gac ggc gtg gat      336
Ser Val Ser Ile Gly Glu Gln Ser Gln Glu Ile Ala Asp Gly Val Asp
            100                 105                 110

aac tcc gac gaa gcc cgc acc aac ggc gac gtt gaa gaa gac gac cgc      384
Asn Ser Asp Glu Ala Arg Thr Asn Gly Asp Val Glu Glu Asp Asp Arg
        115                 120                 125

gca ggt gct ggc gac cag ggc ctg atg ttc ggc tac gcc acc aac gaa      432
Ala Gly Ala Gly Asp Gln Gly Leu Met Phe Gly Tyr Ala Thr Asn Glu
    130                 135                 140

acc gaa gag tac atg cct ctt cct atc gcg ttg gcg cac cga ctg tca 480
Thr Glu Glu Tyr Met Pro Leu Pro Ile Ala Leu Ala His Arg Leu Ser
145                 150                 155                 160

cgt cgt ctg acc cag gtt cgt aaa gag ggc atc gtt cct cac ctg cgt 528
Arg Arg Leu Thr Gln Val Arg Lys Glu Gly Ile Val Pro His Leu Arg
                165                 170                 175

cca gac gga aaa acc cag gtc acc ttc gca tac gat gcg caa gac cgc 576
Pro Asp Gly Lys Thr Gln Val Thr Phe Ala Tyr Asp Ala Gln Asp Arg
            180                 185                 190

cct agc cac ctg gat acc gtt gtc atc tcc acc cag cac gac cca gaa 624
Pro Ser His Leu Asp Thr Val Val Ile Ser Thr Gln His Asp Pro Glu
        195                 200                 205

gtt gac cgt gca tgg ttg gaa acc caa ctg cgc gaa cac gtc att gat 672
Val Asp Arg Ala Trp Leu Glu Thr Gln Leu Arg Glu His Val Ile Asp
    210                 215                 220

tgg gta atc aaa gac gca ggc att gag gat ctg gca acc ggt gag atc 720
Trp Val Ile Lys Asp Ala Gly Ile Glu Asp Leu Ala Thr Gly Glu Ile
225                 230                 235                 240

acc gtg ttg atc aac cct tca ggt tcc ttc att ctg ggt ggc ccc atg 768
Thr Val Leu Ile Asn Pro Ser Gly Ser Phe Ile Leu Gly Gly Pro Met
                245                 250                 255

ggt gat gcg ggt ctg acc ggc cgc aag atc atc gtg gat acc tac ggt 816
Gly Asp Ala Gly Leu Thr Gly Arg Lys Ile Ile Val Asp Thr Tyr Gly
            260                 265                 270

ggc atg gct cgc cat ggt ggt gga gca ttc tcc ggt aag gat cca agc 864
Gly Met Ala Arg His Gly Gly Gly Ala Phe Ser Gly Lys Asp Pro Ser
        275                 280                 285

aag gtg gac cgc tct gct gca tac gcc atg cgt tgg gta gca aag aac 912
Lys Val Asp Arg Ser Ala Ala Tyr Ala Met Arg Trp Val Ala Lys Asn
    290                 295                 300

atc gtg gca gca ggc ctt gct gat cgc gct gaa gtt cag gtt gca tac 960
Ile Val Ala Ala Gly Leu Ala Asp Arg Ala Glu Val Gln Val Ala Tyr
305                 310                 315                 320

gcc att gga cgc gca aag cca gtc gga ctt tac gtt gaa acc ttt gac 1008
Ala Ile Gly Arg Ala Lys Pro Val Gly Leu Tyr Val Glu Thr Phe Asp
                325                 330                 335

acc aac aag gaa ggc ctg agc gac gag cag att cag gct gcc gtg ttg 1056
Thr Asn Lys Glu Gly Leu Ser Asp Glu Gln Ile Gln Ala Ala Val Leu
            340                 345                 350

gag gtc ttt gac ctg cgt cca gca gca att atc cgt gag ctt gat ctg 1104
Glu Val Phe Asp Leu Arg Pro Ala Ala Ile Ile Arg Glu Leu Asp Leu
        355                 360                 365

ctt cgt ccg atc tac gct gac act gct gcc tac ggc cac ttt ggt cgc 1152
Leu Arg Pro Ile Tyr Ala Asp Thr Ala Ala Tyr Gly His Phe Gly Arg
    370                 375                 380

act gat ttg gac ctt cct tgg gag gct atc gac cgc gtt gat gaa ctt 1200
Thr Asp Leu Asp Leu Pro Trp Glu Ala Ile Asp Arg Val Asp Glu Leu
385                 390                 395                 400

cgc gca gcc ctc aag ttg gcc taa 1224
Arg Ala Ala Leu Lys Leu Ala
                405

<210> 2
<211> 407
<212> PRT
<213> Corynebacterium glutamicum

<400> 2

Met Ala Gln Pro Thr Ala Val Arg Leu Phe Thr Ser Glu Ser Val Thr
1               5                   10                  15

Glu Gly His Pro Asp Lys Ile Cys Asp Ala Ile Ser Asp Thr Ile Leu
            20                  25                  30

Asp Ala Leu Leu Glu Lys Asp Pro Gln Ser Arg Val Ala Val Glu Thr
        35                  40                  45

Val Val Thr Thr Gly Ile Val His Val Val Gly Glu Val Arg Thr Ser
    50                  55                  60

Ala Tyr Val Glu Ile Pro Gln Leu Val Arg Asn Lys Leu Ile Glu Ile
65                  70                  75                  80

Gly Phe Asn Ser Ser Glu Val Gly Phe Asp Gly Arg Thr Cys Gly Val
                85                  90                  95

Ser Val Ser Ile Gly Glu Gln Ser Gln Glu Ile Ala Asp Gly Val Asp
            100                 105                 110

Asn Ser Asp Glu Ala Arg Thr Asn Gly Asp Val Glu Glu Asp Asp Arg
        115                 120                 125

Ala Gly Ala Gly Asp Gln Gly Leu Met Phe Gly Tyr Ala Thr Asn Glu
    130                 135                 140

Thr Glu Glu Tyr Met Pro Leu Pro Ile Ala Leu Ala His Arg Leu Ser
145                 150                 155                 160

Arg Arg Leu Thr Gln Val Arg Lys Glu Gly Ile Val Pro His Leu Arg
                165                 170                 175

Pro Asp Gly Lys Thr Gln Val Thr Phe Ala Tyr Asp Ala Gln Asp Arg
            180                 185                 190

Pro Ser His Leu Asp Thr Val Val Ile Ser Thr Gln His Asp Pro Glu
        195                 200                 205

Val Asp Arg Ala Trp Leu Glu Thr Gln Leu Arg Glu His Val Ile Asp
    210                 215                 220

Trp Val Ile Lys Asp Ala Gly Ile Glu Asp Leu Ala Thr Gly Glu Ile
225                 230                 235                 240

Thr Val Leu Ile Asn Pro Ser Gly Ser Phe Ile Leu Gly Gly Pro Met
                245                 250                 255

Gly Asp Ala Gly Leu Thr Gly Arg Lys Ile Ile Val Asp Thr Tyr Gly
            260                 265                 270

Gly Met Ala Arg His Gly Gly Gly Ala Phe Ser Gly Lys Asp Pro Ser
        275                 280                 285

Lys Val Asp Arg Ser Ala Ala Tyr Ala Met Arg Trp Val Ala Lys Asn
    290                 295                 300

Ile Val Ala Ala Gly Leu Ala Asp Arg Ala Glu Val Gln Val Ala Tyr
305                 310                 315                 320

Ala Ile Gly Arg Ala Lys Pro Val Gly Leu Tyr Val Glu Thr Phe Asp
                325                 330                 335

Thr Asn Lys Glu Gly Leu Ser Asp Glu Gln Ile Gln Ala Ala Val Leu
            340                 345                 350

Glu Val Phe Asp Leu Arg Pro Ala Ala Ile Ile Arg Glu Leu Asp Leu
        355                 360                 365

Leu Arg Pro Ile Tyr Ala Asp Thr Ala Ala Tyr Gly His Phe Gly Arg
    370                 375                 380

Thr Asp Leu Asp Leu Pro Trp Glu Ala Ile Asp Arg Val Asp Glu Leu
385                 390                 395                 400

Arg Ala Ala Leu Lys Leu Ala
                405

<210>  3
<211>  1224

<212> DNA
<213> Corynebacterium glutamicum

<220>
<221> CDS
<222> (1)..(1221)
<223> metK allele

<220>
<221> mutation
<222> (599)..(599)
<223> Transversion: thymine--> adenine

<400> 3
gtg gct cag cca acc gcc gtc cgt ttg ttc acc agt gaa tct gta act 48
Met Ala Gln Pro Thr Ala Val Arg Leu Phe Thr Ser Glu Ser Val Thr
1               5                   10                  15

gag gga cat cca gac aaa ata tgt gat gct att tcc gat acc att ttg 96
Glu Gly His Pro Asp Lys Ile Cys Asp Ala Ile Ser Asp Thr Ile Leu
            20                  25                  30

gac gcg ctg ctc gaa aaa gat ccg cag tcg cgc gtc gca gtg gaa act 144
Asp Ala Leu Leu Glu Lys Asp Pro Gln Ser Arg Val Ala Val Glu Thr
        35                  40                  45

gtg gtc acc acc gga atc gtc cat gtt gtt ggc gag gtc cgt acc agc 192
Val Val Thr Thr Gly Ile Val His Val Val Gly Glu Val Arg Thr Ser
    50                  55                  60

gct tac gta gag atc cct caa tta gtc cgc aac aag ctc atc gaa atc 240
Ala Tyr Val Glu Ile Pro Gln Leu Val Arg Asn Lys Leu Ile Glu Ile
65                  70                  75                  80

gga ttc aac tcc tct gag gtt gga ttc gac gga cgc acc tgt ggc gtc 288
Gly Phe Asn Ser Ser Glu Val Gly Phe Asp Gly Arg Thr Cys Gly Val
                85                  90                  95

tca gta tcc atc ggt gag cag tcc cag gaa atc gct gac ggc gtg gat 336
Ser Val Ser Ile Gly Glu Gln Ser Gln Glu Ile Ala Asp Gly Val Asp
            100                 105                 110

aac tcc gac gaa gcc cgc acc aac ggc gac gtt gaa gaa gac gac cgc 384
Asn Ser Asp Glu Ala Arg Thr Asn Gly Asp Val Glu Glu Asp Asp Arg
        115                 120                 125

gca ggt gct ggc gac cag ggc ctg atg ttc ggc tac gcc acc aac gaa 432
Ala Gly Ala Gly Asp Gln Gly Leu Met Phe Gly Tyr Ala Thr Asn Glu
    130                 135                 140

acc gaa gag tac atg cct ctt cct atc gcg ttg gcg cac cga ctg tca 480
Thr Glu Glu Tyr Met Pro Leu Pro Ile Ala Leu Ala His Arg Leu Ser
145                 150                 155                 160

cgt cgt ctg acc cag gtt cgt aaa gag ggc atc gtt cct cac ctg cgt 528
Arg Arg Leu Thr Gln Val Arg Lys Glu Gly Ile Val Pro His Leu Arg
                165                 170                 175

cca gac gga aaa acc cag gtc acc ttc gca tac gat gcg caa gac cgc 576
Pro Asp Gly Lys Thr Gln Val Thr Phe Ala Tyr Asp Ala Gln Asp Arg
            180                 185                 190

cct agc cac ctg gat acc gtt gac atc tcc acc cag cac gac cca gaa 624
Pro Ser His Leu Asp Thr Val Asp Ile Ser Thr Gln His Asp Pro Glu
        195                 200                 205

gtt gac cgt gca tgg ttg gaa acc caa ctg cgc gaa cac gtc att gat 672
Val Asp Arg Ala Trp Leu Glu Thr Gln Leu Arg Glu His Val Ile Asp
    210                 215                 220

tgg gta atc aaa gac gca ggc att gag gat ctg gca acc ggt gag atc 720
Trp Val Ile Lys Asp Ala Gly Ile Glu Asp Leu Ala Thr Gly Glu Ile
225                 230                 235                 240

acc gtg ttg atc aac cct tca ggt tcc ttc att ctg ggt ggc ccc atg 768
Thr Val Leu Ile Asn Pro Ser Gly Ser Phe Ile Leu Gly Gly Pro Met
                245                 250                 255

ggt gat gcg ggt ctg acc ggc cgc aag atc atc gtg gat acc tac ggt 816
Gly Asp Ala Gly Leu Thr Gly Arg Lys Ile Ile Val Asp Thr Tyr Gly
            260                 265                 270

ggc atg gct cgc cat ggt ggt gga gca ttc tcc ggt aag gat cca agc 864
Gly Met Ala Arg His Gly Gly Gly Ala Phe Ser Gly Lys Asp Pro Ser
        275                 280                 285

aag gtg gac cgc tct gct gca tac gcc atg cgt tgg gta gca aag aac 912
Lys Val Asp Arg Ser Ala Ala Tyr Ala Met Arg Trp Val Ala Lys Asn
    290                 295                 300

atc gtg gca gca ggc ctt gct gat cgc gct gaa gtt cag gtt gca tac 960
Ile Val Ala Ala Gly Leu Ala Asp Arg Ala Glu Val Gln Val Ala Tyr
305                 310                 315                 320

gcc att gga cgc gca aag cca gtc gga ctt tac gtt gaa acc ttt gac 1008
Ala Ile Gly Arg Ala Lys Pro Val Gly Leu Tyr Val Glu Thr Phe Asp
                325                 330                 335

acc aac aag gaa ggc ctg agc gac gag cag att cag gct gcc gtg ttg 1056
Thr Asn Lys Glu Gly Leu Ser Asp Glu Gln Ile Gln Ala Ala Val Leu
            340                 345                 350

gag gtc ttt gac ctg cgt cca gca gca att atc cgt gag ctt gat ctg 1104
Glu Val Phe Asp Leu Arg Pro Ala Ala Ile Ile Arg Glu Leu Asp Leu
        355                 360                 365

ctt cgt ccg atc tac gct gac act gct gcc tac ggc cac ttt ggt cgc 1152
Leu Arg Pro Ile Tyr Ala Asp Thr Ala Ala Tyr Gly His Phe Gly Arg
    370                 375                 380

act gat ttg gac ctt cct tgg gag gct atc gac cgc gtt gat gaa ctt 1200
Thr Asp Leu Asp Leu Pro Trp Glu Ala Ile Asp Arg Val Asp Glu Leu
385                 390                 395                 400

cgc gca gcc ctc aag ttg gcc taa 1224
Arg Ala Ala Leu Lys Leu Ala
                405

<210> 4
<211> 407
<212> PRT
<213> Corynebacterium glutamicum

<400> 4

Met Ala Gln Pro Thr Ala Val Arg Leu Phe Thr Ser Glu Ser Val Thr
1               5                   10                  15

Glu Gly His Pro Asp Lys Ile Cys Asp Ala Ile Ser Asp Thr Ile Leu
            20                  25                  30

Asp Ala Leu Leu Glu Lys Asp Pro Gln Ser Arg Val Ala Val Glu Thr
        35                  40                  45

Val Val Thr Thr Gly Ile Val His Val Val Gly Glu Val Arg Thr Ser
    50                  55                  60

Ala Tyr Val Glu Ile Pro Gln Leu Val Arg Asn Lys Leu Ile Glu Ile
65                  70                  75                  80

Gly Phe Asn Ser Ser Glu Val Gly Phe Asp Gly Arg Thr Cys Gly Val
                85                  90                  95

Ser Val Ser Ile Gly Glu Gln Ser Gln Glu Ile Ala Asp Gly Val Asp
            100                 105                 110

Asn Ser Asp Glu Ala Arg Thr Asn Gly Asp Val Glu Glu Asp Asp Arg
        115                 120                 125

Ala Gly Ala Gly Asp Gln Gly Leu Met Phe Gly Tyr Ala Thr Asn Glu
    130                 135                 140

Thr Glu Glu Tyr Met Pro Leu Pro Ile Ala Leu Ala His Arg Leu Ser
145                 150                 155                 160

Arg Arg Leu Thr Gln Val Arg Lys Glu Gly Ile Val Pro His Leu Arg
                165                 170                 175

Pro Asp Gly Lys Thr Gln Val Thr Phe Ala Tyr Asp Ala Gln Asp Arg
            180                 185                 190

Pro Ser His Leu Asp Thr Val Asp Ile Ser Thr Gln His Asp Pro Glu
        195                 200                 205

Val Asp Arg Ala Trp Leu Glu Thr Gln Leu Arg Glu His Val Ile Asp
    210                 215                 220

Trp Val Ile Lys Asp Ala Gly Ile Glu Asp Leu Ala Thr Gly Glu Ile
225                 230                 235                 240

Thr Val Leu Ile Asn Pro Ser Gly Ser Phe Ile Leu Gly Gly Pro Met
                245                 250                 255

Gly Asp Ala Gly Leu Thr Gly Arg Lys Ile Ile Val Asp Thr Tyr Gly
            260                 265                 270

Gly Met Ala Arg His Gly Gly Gly Ala Phe Ser Gly Lys Asp Pro Ser
        275                 280                 285

Lys Val Asp Arg Ser Ala Ala Tyr Ala Met Arg Trp Val Ala Lys Asn
    290                 295                 300

Ile Val Ala Ala Gly Leu Ala Asp Arg Ala Glu Val Gln Val Ala Tyr
305                 310                 315                 320

Ala Ile Gly Arg Ala Lys Pro Val Gly Leu Tyr Val Glu Thr Phe Asp
                325                 330                 335

Thr Asn Lys Glu Gly Leu Ser Asp Glu Gln Ile Gln Ala Ala Val Leu
            340                 345                 350

Glu Val Phe Asp Leu Arg Pro Ala Ala Ile Ile Arg Glu Leu Asp Leu
        355                 360                 365

Leu Arg Pro Ile Tyr Ala Asp Thr Ala Ala Tyr Gly His Phe Gly Arg
    370                 375                 380

Thr Asp Leu Asp Leu Pro Trp Glu Ala Ile Asp Arg Val Asp Glu Leu
385                 390                 395                 400

Arg Ala Ala Leu Lys Leu Ala
                405

<210>  5
<211>  1224
<212>  DNA
<213>  Corynebacterium glutamicum

<220>
<221>  CDS
<222>  (1)..(1221)
<223>  metK allele

<220>
<221>  mutation
<222>  (964)..(964)
<223>  Transversion: adenine --> cytosine

<400> 5
gtg gct cag cca acc gcc gtc cgt ttg ttc acc agt gaa tct gta act 48
Met Ala Gln Pro Thr Ala Val Arg Leu Phe Thr Ser Glu Ser Val Thr
1               5                   10                  15

gag gga cat cca gac aaa ata tgt gat gct att tcc gat acc att ttg 96
Glu Gly His Pro Asp Lys Ile Cys Asp Ala Ile Ser Asp Thr Ile Leu
            20                  25                  30

gac gcg ctg ctc gaa aaa gat ccg cag tcg cgc gtc gca gtg gaa act 144
Asp Ala Leu Leu Glu Lys Asp Pro Gln Ser Arg Val Ala Val Glu Thr
        35                  40                  45

gtg gtc acc acc gga atc gtc cat gtt gtt ggc gag gtc cgt acc agc 192
Val Val Thr Thr Gly Ile Val His Val Val Gly Glu Val Arg Thr Ser
    50                  55                  60

gct tac gta gag atc cct caa tta gtc cgc aac aag ctc atc gaa atc 240
Ala Tyr Val Glu Ile Pro Gln Leu Val Arg Asn Lys Leu Ile Glu Ile
65                  70                  75                  80

gga ttc aac tcc tct gag gtt gga ttc gac gga cgc acc tgt ggc gtc 288
Gly Phe Asn Ser Ser Glu Val Gly Phe Asp Gly Arg Thr Cys Gly Val
                85                  90                  95

tca gta tcc atc ggt gag cag tcc cag gaa atc gct gac ggc gtg gat 336
Ser Val Ser Ile Gly Glu Gln Ser Gln Glu Ile Ala Asp Gly Val Asp
            100                 105                 110

aac tcc gac gaa gcc cgc acc aac ggc gac gtt gaa gaa gac gac cgc 384
Asn Ser Asp Glu Ala Arg Thr Asn Gly Asp Val Glu Glu Asp Asp Arg
        115                 120                 125

gca ggt gct ggc gac cag ggc ctg atg ttc ggc tac gcc acc aac gaa 432
Ala Gly Ala Gly Asp Gln Gly Leu Met Phe Gly Tyr Ala Thr Asn Glu
    130                 135                 140

acc gaa gag tac atg cct ctt cct atc gcg ttg gcg cac cga ctg tca 480
Thr Glu Glu Tyr Met Pro Leu Pro Ile Ala Leu Ala His Arg Leu Ser
145                 150                 155                 160

cgt cgt ctg acc cag gtt cgt aaa gag ggc atc gtt cct cac ctg cgt 528
Arg Arg Leu Thr Gln Val Arg Lys Glu Gly Ile Val Pro His Leu Arg
                165                 170                 175

cca gac gga aaa acc cag gtc acc ttc gca tac gat gcg caa gac cgc 576
Pro Asp Gly Lys Thr Gln Val Thr Phe Ala Tyr Asp Ala Gln Asp Arg
            180                 185                 190

cct agc cac ctg gat acc gtt gtc atc tcc acc cag cac gac cca gaa 624
Pro Ser His Leu Asp Thr Val Val Ile Ser Thr Gln His Asp Pro Glu
        195                 200                 205

gtt gac cgt gca tgg ttg gaa acc caa ctg cgc gaa cac gtc att gat 672
Val Asp Arg Ala Trp Leu Glu Thr Gln Leu Arg Glu His Val Ile Asp
    210                 215                 220

tgg gta atc aaa gac gca ggc att gag gat ctg gca acc ggt gag atc 720
Trp Val Ile Lys Asp Ala Gly Ile Glu Asp Leu Ala Thr Gly Glu Ile
225                 230                 235                 240

acc gtg ttg atc aac cct tca ggt tcc ttc att ctg ggt ggc ccc atg 768
Thr Val Leu Ile Asn Pro Ser Gly Ser Phe Ile Leu Gly Gly Pro Met
                245                 250                 255

ggt gat gcg ggt ctg acc ggc cgc aag atc atc gtg gat acc tac ggt 816
Gly Asp Ala Gly Leu Thr Gly Arg Lys Ile Ile Val Asp Thr Tyr Gly
            260                 265                 270

ggc atg gct cgc cat ggt ggt gga gca ttc tcc ggt aag gat cca agc 864
Gly Met Ala Arg His Gly Gly Gly Ala Phe Ser Gly Lys Asp Pro Ser
        275                 280                 285

aag gtg gac cgc tct gct gca tac gcc atg cgt tgg gta gca aag aac 912
Lys Val Asp Arg Ser Ala Ala Tyr Ala Met Arg Trp Val Ala Lys Asn
    290                 295                 300

atc gtg gca gca ggc ctt gct gat cgc gct gaa gtt cag gtt gca tac 960
Ile Val Ala Ala Gly Leu Ala Asp Arg Ala Glu Val Gln Val Ala Tyr
305                 310                 315                 320

gcc ctt gga cgc gca aag cca gtc gga ctt tac gtt gaa acc ttt gac 1008
Ala Leu Gly Arg Ala Lys Pro Val Gly Leu Tyr Val Glu Thr Phe Asp
                325                 330                 335

acc aac aag gaa ggc ctg agc gac gag cag att cag gct gcc gtg ttg 1056
Thr Asn Lys Glu Gly Leu Ser Asp Glu Gln Ile Gln Ala Ala Val Leu
            340                 345                 350

gag gtc ttt gac ctg cgt cca gca gca att atc cgt gag ctt gat ctg 1104
Glu Val Phe Asp Leu Arg Pro Ala Ala Ile Ile Arg Glu Leu Asp Leu
        355                 360                 365

ctt cgt ccg atc tac gct gac act gct gcc tac ggc cac ttt ggt cgc 1152
Leu Arg Pro Ile Tyr Ala Asp Thr Ala Ala Tyr Gly His Phe Gly Arg
    370                 375                 380

act gat ttg gac ctt cct tgg gag gct atc gac cgc gtt gat gaa ctt 1200
Thr Asp Leu Asp Leu Pro Trp Glu Ala Ile Asp Arg Val Asp Glu Leu
385                 390                 395                 400

cgc gca gcc ctc aag ttg gcc taa 1224
Arg Ala Ala Leu Lys Leu Ala
                405

<210> 6
<211> 407
<212> PRT
<213> Corynebacterium glutamicum

<400> 6

Met Ala Gln Pro Thr Ala Val Arg Leu Phe Thr Ser Glu Ser Val Thr
1               5                   10                  15

Glu Gly His Pro Asp Lys Ile Cys Asp Ala Ile Ser Asp Thr Ile Leu
            20                  25                  30

Asp Ala Leu Leu Glu Lys Asp Pro Gln Ser Arg Val Ala Val Glu Thr
        35                  40                  45

Val Val Thr Thr Gly Ile Val His Val Val Gly Glu Val Arg Thr Ser
    50                  55                  60

Ala Tyr Val Glu Ile Pro Gln Leu Val Arg Asn Lys Leu Ile Glu Ile
65                  70                  75                  80

Gly Phe Asn Ser Ser Glu Val Gly Phe Asp Gly Arg Thr Cys Gly Val
                85                  90                  95

Ser Val Ser Ile Gly Glu Gln Ser Gln Glu Ile Ala Asp Gly Val Asp
            100                 105                 110

Asn Ser Asp Glu Ala Arg Thr Asn Gly Asp Val Glu Glu Asp Asp Arg
        115                 120                 125

Ala Gly Ala Gly Asp Gln Gly Leu Met Phe Gly Tyr Ala Thr Asn Glu
    130                 135                 140

Thr Glu Glu Tyr Met Pro Leu Pro Ile Ala Leu Ala His Arg Leu Ser
145                 150                 155                 160

Arg Arg Leu Thr Gln Val Arg Lys Glu Gly Ile Val Pro His Leu Arg
                165                 170                 175

Pro Asp Gly Lys Thr Gln Val Thr Phe Ala Tyr Asp Ala Gln Asp Arg
            180                 185                 190

Pro Ser His Leu Asp Thr Val Val Ile Ser Thr Gln His Asp Pro Glu
        195                 200                 205

Val Asp Arg Ala Trp Leu Glu Thr Gln Leu Arg Glu His Val Ile Asp
    210                 215                 220

Trp Val Ile Lys Asp Ala Gly Ile Glu Asp Leu Ala Thr Gly Glu Ile
225                 230                 235                 240

Thr Val Leu Ile Asn Pro Ser Gly Ser Phe Ile Leu Gly Gly Pro Met
                245                 250                 255

Gly Asp Ala Gly Leu Thr Gly Arg Lys Ile Ile Val Asp Thr Tyr Gly
            260                 265                 270

Gly Met Ala Arg His Gly Gly Gly Ala Phe Ser Gly Lys Asp Pro Ser
        275                 280                 285

Lys Val Asp Arg Ser Ala Ala Tyr Ala Met Arg Trp Val Ala Lys Asn
    290                 295                 300

Ile Val Ala Ala Gly Leu Ala Asp Arg Ala Glu Val Gln Val Ala Tyr
305                 310                 315                 320

Ala Leu Gly Arg Ala Lys Pro Val Gly Leu Tyr Val Glu Thr Phe Asp
                325                 330                 335

Thr Asn Lys Glu Gly Leu Ser Asp Glu Gln Ile Gln Ala Ala Val Leu
            340                 345                 350

Glu Val Phe Asp Leu Arg Pro Ala Ala Ile Ile Arg Glu Leu Asp Leu
        355                 360                 365

Leu Arg Pro Ile Tyr Ala Asp Thr Ala Ala Tyr Gly His Phe Gly Arg
    370                 375                 380

Thr Asp Leu Asp Leu Pro Trp Glu Ala Ile Asp Arg Val Asp Glu Leu
385                 390                 395                 400

Arg Ala Ala Leu Lys Leu Ala
                405

<210>  7
<211>  1236
<212>  DNA
<213>  Corynebacterium glutamicum

<220>
<221>  CDS
<222>  (1)..(1233)
<223>  metK allele

<220>
<221>  mutation
<222>  (1201)..(1201)
<223>  frame shift mutation: deletion of the base cytosine

<400>  7

gtg gct cag cca acc gcc gtc cgt ttg ttc acc agt gaa tct gta act        48
Met Ala Gln Pro Thr Ala Val Arg Leu Phe Thr Ser Glu Ser Val Thr
1               5                   10                  15

gag gga cat cca gac aaa ata tgt gat gct att tcc gat acc att ttg        96
Glu Gly His Pro Asp Lys Ile Cys Asp Ala Ile Ser Asp Thr Ile Leu
            20                  25                  30

gac gcg ctg ctc gaa aaa gat ccg cag tcg cgc gtc gca gtg gaa act 144
Asp Ala Leu Leu Glu Lys Asp Pro Gln Ser Arg Val Ala Val Glu Thr
        35                  40                  45

gtg gtc acc acc gga atc gtc cat gtt gtt ggc gag gtc cgt acc agc 192
Val Val Thr Thr Gly Ile Val His Val Val Gly Glu Val Arg Thr Ser
    50                  55                  60

gct tac gta gag atc cct caa tta gtc cgc aac aag ctc atc gaa atc 240
Ala Tyr Val Glu Ile Pro Gln Leu Val Arg Asn Lys Leu Ile Glu Ile
65                  70                  75                  80

gga ttc aac tcc tct gag gtt gga ttc gac gga cgc acc tgt ggc gtc 288
Gly Phe Asn Ser Ser Glu Val Gly Phe Asp Gly Arg Thr Cys Gly Val
                85                  90                  95

tca gta tcc atc ggt gag cag tcc cag gaa atc gct gac ggc gtg gat 336
Ser Val Ser Ile Gly Glu Gln Ser Gln Glu Ile Ala Asp Gly Val Asp
            100                 105                 110

aac tcc gac gaa gcc cgc acc aac ggc gac gtt gaa gaa gac gac cgc 384
Asn Ser Asp Glu Ala Arg Thr Asn Gly Asp Val Glu Glu Asp Asp Arg
        115                 120                 125

gca ggt gct ggc gac cag ggc ctg atg ttc ggc tac gcc acc aac gaa 432
Ala Gly Ala Gly Asp Gln Gly Leu Met Phe Gly Tyr Ala Thr Asn Glu
    130                 135                 140

acc gaa gag tac atg cct ctt cct atc gcg ttg gcg cac cga ctg tca 480
Thr Glu Glu Tyr Met Pro Leu Pro Ile Ala Leu Ala His Arg Leu Ser
145                 150                 155                 160

cgt cgt ctg acc cag gtt cgt aaa gag ggc atc gtt cct cac ctg cgt 528
Arg Arg Leu Thr Gln Val Arg Lys Glu Gly Ile Val Pro His Leu Arg
                165                 170                 175

cca gac gga aaa acc cag gtc acc ttc gca tac gat gcg caa gac cgc 576
Pro Asp Gly Lys Thr Gln Val Thr Phe Ala Tyr Asp Ala Gln Asp Arg
            180                 185                 190

cct agc cac ctg gat acc gtt gtc atc tcc acc cag cac gac cca gaa 624
Pro Ser His Leu Asp Thr Val Val Ile Ser Thr Gln His Asp Pro Glu
        195                 200                 205

gtt gac cgt gca tgg ttg gaa acc caa ctg cgc gaa cac gtc att gat 672
Val Asp Arg Ala Trp Leu Glu Thr Gln Leu Arg Glu His Val Ile Asp
    210                 215                 220

tgg gta atc aaa gac gca ggc att gag gat ctg gca acc ggt gag atc 720
Trp Val Ile Lys Asp Ala Gly Ile Glu Asp Leu Ala Thr Gly Glu Ile
225                 230                 235                 240

acc gtg ttg atc aac cct tca ggt tcc ttc att ctg ggt ggc ccc atg 768
Thr Val Leu Ile Asn Pro Ser Gly Ser Phe Ile Leu Gly Gly Pro Met
                245                 250                 255

ggt gat gcg ggt ctg acc ggc cgc aag atc atc gtg gat acc tac ggt 816
Gly Asp Ala Gly Leu Thr Gly Arg Lys Ile Ile Val Asp Thr Tyr Gly
            260                 265                 270

ggc atg gct cgc cat ggt ggt gga gca ttc tcc ggt aag gat cca agc 864
Gly Met Ala Arg His Gly Gly Gly Ala Phe Ser Gly Lys Asp Pro Ser
        275                 280                 285

aag gtg gac cgc tct gct gca tac gcc atg cgt tgg gta gca aag aac 912
Lys Val Asp Arg Ser Ala Ala Tyr Ala Met Arg Trp Val Ala Lys Asn
    290                 295                 300

atc gtg gca gca ggc ctt gct gat cgc gct gaa gtt cag gtt gca tac 960
Ile Val Ala Ala Gly Leu Ala Asp Arg Ala Glu Val Gln Val Ala Tyr
305                 310                 315                 320

gcc att gga cgc gca aag cca gtc gga ctt tac gtt gaa acc ttt gac 1008
Ala Ile Gly Arg Ala Lys Pro Val Gly Leu Tyr Val Glu Thr Phe Asp
                325                 330                 335

acc aac aag gaa ggc ctg agc gac gag cag att cag gct gcc gtg ttg 1056
Thr Asn Lys Glu Gly Leu Ser Asp Glu Gln Ile Gln Ala Ala Val Leu
            340                 345                 350

gag gtc ttt gac ctg cgt cca gca gca att atc cgt gag ctt gat ctg 1104
Glu Val Phe Asp Leu Arg Pro Ala Ala Ile Ile Arg Glu Leu Asp Leu
        355                 360                 365

ctt cgt ccg atc tac gct gac act gct gcc tac ggc cac ttt ggt cgc 1152
Leu Arg Pro Ile Tyr Ala Asp Thr Ala Ala Tyr Gly His Phe Gly Arg
    370                 375                 380

act gat ttg gac ctt cct tgg gag gct atc gac cgc gtt gat gaa ctt 1200
Thr Asp Leu Asp Leu Pro Trp Glu Ala Ile Asp Arg Val Asp Glu Leu
385                 390                 395                 400

gcg cag ccc tca agt tgg cct aaa aat ctg atg tag 1236
Ala Gln Pro Ser Ser Trp Pro Lys Asn Leu Met
                405                 410

<210> 8
<211> 411
<212> PRT
<213> Corynebacterium glutamicum

<400> 8

Met Ala Gln Pro Thr Ala Val Arg Leu Phe Thr Ser Glu Ser Val Thr
1               5                   10                  15

Glu Gly His Pro Asp Lys Ile Cys Asp Ala Ile Ser Asp Thr Ile Leu
            20                  25                  30

Asp Ala Leu Leu Glu Lys Asp Pro Gln Ser Arg Val Ala Val Glu Thr
        35                  40                  45

Val Val Thr Thr Gly Ile Val His Val Val Gly Glu Val Arg Thr Ser
    50                  55                  60

Ala Tyr Val Glu Ile Pro Gln Leu Val Arg Asn Lys Leu Ile Glu Ile
65                  70                  75                  80

Gly Phe Asn Ser Ser Glu Val Gly Phe Asp Gly Arg Thr Cys Gly Val
                85                  90                  95

Ser Val Ser Ile Gly Glu Gln Ser Gln Glu Ile Ala Asp Gly Val Asp
            100                 105                 110

Asn Ser Asp Glu Ala Arg Thr Asn Gly Asp Val Glu Glu Asp Asp Arg
        115                 120                 125

Ala Gly Ala Gly Asp Gln Gly Leu Met Phe Gly Tyr Ala Thr Asn Glu
    130                 135                 140

Thr Glu Glu Tyr Met Pro Leu Pro Ile Ala Leu Ala His Arg Leu Ser
145                 150                 155                 160

Arg Arg Leu Thr Gln Val Arg Lys Glu Gly Ile Val Pro His Leu Arg
                165                 170                 175

Pro Asp Gly Lys Thr Gln Val Thr Phe Ala Tyr Asp Ala Gln Asp Arg
            180                 185                 190

Pro Ser His Leu Asp Thr Val Val Ile Ser Thr Gln His Asp Pro Glu
        195                 200                 205

Val Asp Arg Ala Trp Leu Glu Thr Gln Leu Arg Glu His Val Ile Asp
    210                 215                 220

Trp Val Ile Lys Asp Ala Gly Ile Glu Asp Leu Ala Thr Gly Glu Ile
225                 230                 235                 240

Thr Val Leu Ile Asn Pro Ser Gly Ser Phe Ile Leu Gly Gly Pro Met
                245                 250                 255

Gly Asp Ala Gly Leu Thr Gly Arg Lys Ile Ile Val Asp Thr Tyr Gly
            260                 265                 270

Gly Met Ala Arg His Gly Gly Gly Ala Phe Ser Gly Lys Asp Pro Ser
        275                 280                 285

Lys Val Asp Arg Ser Ala Ala Tyr Ala Met Arg Trp Val Ala Lys Asn
    290                 295                 300

Ile Val Ala Ala Gly Leu Ala Asp Arg Ala Glu Val Gln Val Ala Tyr
305                 310                 315                 320

Ala Ile Gly Arg Ala Lys Pro Val Gly Leu Tyr Val Glu Thr Phe Asp
                325                 330                 335

Thr Asn Lys Glu Gly Leu Ser Asp Glu Gln Ile Gln Ala Ala Val Leu
            340                 345                 350

Glu Val Phe Asp Leu Arg Pro Ala Ala Ile Ile Arg Glu Leu Asp Leu
        355                 360                 365

Leu Arg Pro Ile Tyr Ala Asp Thr Ala Ala Tyr Gly His Phe Gly Arg
    370                 375                 380

Thr Asp Leu Asp Leu Pro Trp Glu Ala Ile Asp Arg Val Asp Glu Leu
385                 390                 395                 400

Ala Gln Pro Ser Ser Trp Pro Lys Asn Leu Met
                405                 410

<210>  9
<211>  30
<212>  DNA
<213>  Artificial sequence

<220>
<223>  Primer ccpA1-del1

<220>
<221>  misc_feature
<222>  (1)..(30)
<223>  Primer ccpA1-del1

<400>  9
gctagaattc aaggccacat tgaccagttc                                        30

<210>  10
<211>  48
<212>  DNA
<213>  Artificial sequence

<220>
<223>  Primer ccpA1-del2

<220>
<221>  misc_feature
<222>  (1)..(48)
<223>  Primer ccpA1-del2

<400>  10
tgtgacctag gcttgacttt atttaaatat gatcctgcac caatcctt                    48

<210> 11
<211> 48
<212> DNA
<213> Artificial sequence

<220>
<223> Primer ccpA1-del3


<220>
<221> misc_feature
<222> (1)..(48)
<223> Primer ccpA1-del3

<400> 11
aaggattggt gcaggatcat atttaaataa agtcaagcct aggtcaca 48


<210> 12
<211> 30
<212> DNA
<213> Artificial sequence

<220>
<223> Primer ccpA1-del4


<220>
<221> misc_feature
<222> (1)..(30)
<223> Primer ccpA1-del4

<400> 12
gctagaattc atctcatcca agtggtgcga 30


<210> 13
<211> 20
<212> DNA
<213> Corynebacterium glutamicum


<220>
<221> misc_feature
<222> (1)..(20)
<223> Primer ccpA1-A1

<400> 13
cctgcacacc accgctgaat 20


<210> 14
<211> 20
<212> DNA
<213> Corynebacterium glutamicum


<220>
<221> misc_feature
<222> (1)..(20)
<223> Primer ccpA1-E1

<400>  14
cagccgcacc agctcatctt                                                    20


<210>  15
<211>  20
<212>  DNA
<213>  Corynebacterium glutamicum


<220>
<221>  misc_feature
<222>  (1)..(20)
<223>  Primer metK-1

<400>  15
tgcgacctcc tcatgtgtaa                                                    20


<210>  16
<211>  20
<212>  DNA
<213>  Corynebacterium glutamicum


<220>
<221>  misc_feature
<222>  (1)..(20)
<223>  Primer metK-2

<400>  16
aggttgtcgt cgccatcatt                                                    20


<210>  17
<211>  30
<212>  DNA
<213>  Corynebacterium glutamicum


<220>
<221>  misc_feature
<222>  (1)..(30)
<223>  Primer metK-I322La

<400>  17
caggttgcat acgcccttgg acgcgcaaag                                         30


<210>  18
<211>  30
<212>  DNA
<213>  Corynebacterium glutamicum


<220>
<221>  misc_feature
<222>  (1)..(30)
<223>  Primer metK-I322Lb

<400> 18
ctttgcgcgt ccaagggcgt atgcaacctg 30

## Beschreibung

Gegenstand der Erfindung sind Allele des metK-Gens aus coryneformen Bakterien kodierend für Varianten des S-Adenosylmethionin-Synthetase (EC: 2.5.1.6) und Verfahren zur fermentativen Herstellung von L-Methionin unter Verwendung von Bakterien, die diese Allele enthalten.

### Stand der Technik

Chemische Verbindungen, mit denen insbesondere L-Aminosäuren, Vitamine, Nukleoside und Nukleotide und D-Aminosäuren gemeint sind, finden in der Humanmedizin, in der pharmazeutischen Industrie, in der Kosmetik, in der Lebensmittelindustrie und in der Tierernährung Anwendung.

Es ist bekannt, dass zahlreiche dieser Verbindungen durch Fermentation von Stämmen coryneformer Bakterien, insbesondere Corynebacterium glutamicum, hergestellt werden. Wegen der großen Bedeutung wird ständig an der Verbesserung der Herstellverfahren gearbeitet. Verfahrensverbesserungen können fermentationstechnische Maßnahmen wie zum Beispiel Rührung und Versorgung mit Sauerstoff, oder die Zusammensetzung der Nährmedien, wie zum Beispiel die Zuckerkonzentration während der Fermentation, oder die Aufarbeitung zur Produktform durch zum Beispiel Ionenaustauschchromatographie oder die intrinsischen Leistungseigenschaften des Mikroorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite oder auxotroph für regulatorisch bedeutsame Metabolite sind und die Aminosäuren produzieren. Bekannte Antimetabolite sind z.B. das Lysin-Analogon S-(2-Aminoethyl)-Cystein oder die Methionin-Analoga α-Methyl-Methionin, Ethionin, Norleucin, N-acetylnorleucin, S-Trifluoromethylhomocystein, 2-amino-5-heptensäure, Seleno-Methionin, Methioninsulfoximin, Methoxin und 1-Aminocyclopentan-Carboxylsäure.

Seit einigen Jahren werden ebenfalls Methoden der rekombinanten DNA-Technik zur Stammverbesserung von L-Aminosäure produzierenden Stämmen von Corynebacterium eingesetzt, indem man einzelne Aminosäure-Biosynthesegene amplifiziert und die Auswirkung auf die Aminosäure-Produktion untersucht.

Die Nukleotidsequenz des für die S-Adenosylmethionin-Synthetase von Corynebacterium glutamicum kodierenden metK-Gens ist in der Genbank unter der Accession Number AJ290443 hinterlegt. Sie kann weiterhin der Patentanmeldung WO01/00843 unter dem Identification Code AX063959 als SEQ ID No. 241 entnommen werden.

Das Gen metK kodiert für die S-Adenosylmethionin-Synthetase (EC: 2.5.1.6) in coryneformen Bakterien, welche den Abbau von Methionin zu S-Adenosylmethionin (SAM) unter verbrauch von ATP katalysiert (Großmann et al., FEMS Microbiology Letters 193: 99-102 (2000)). Die Nukleotidsequenz ist in SEQ ID No. 1 dargestellt, das zugehörige Protein ist in SEQ ID No. 2 dargestellt. von der Zelle gebildetes Methionin wird entweder in der Proteinbiosynthese verbraucht oder durch die S-Adenosylmethionin-Synthetase zu SAM gespalten. SAM stellt den Haupt-Methylgruppendonor der Zelle dar (Cantoni in: Salvatore et al., The Biochemistry of Adenosylmethionine. Columbia University Press, New York: 557 - 577 (1977)). Weiterhin wirkt es inhibierend auf die an der Methionin-Biosynthese beteiligten Genprodukte der Gene metB, kodierend für die Cystathionin-Gamma-Synthase (Kase und Nakayama, Agricultural and Biological Chemistry 38(11): 2235 - 2242 (1974)), sowie metx, kodierend für die Homoserin-Acetyl-Transferase, und metY, kodierend für die O-Acetylhomoserin-Sulfhydrylase (Rey, Diplomarbeit an der Fakultät für Biologie der Universität Bielefeld, 2000).

Für die Produktion von L-Methionin wäre es deshalb wünschenswert, Bakterien einzusetzen, in denen die S-Adenosylmethionin-Synthetase nicht aktiv oder in ihrer Aktivität abgeschwächt ist und/oder die Expression des metK-Gens herabgesetzt ist.

Mutationen, die die Aktivität von MetK in Escherichia coli herabsetzen, führen zu massiven Störungen in der Zellteilung, DNA-Methylierung und Fettsäure-Biosynthese (Greene et al., Journal of Bacteriology 115(1): 57 - 67 (1973) und Newman et al., Journal of Bacteriology 180(14): 3614 - 3619 (1998). Von Wei und Newman (Molecular Microbiology 43(6): 1651-1656 (2002)) wird metK als essentielles Gen für Escherichia coli K12 beschrieben, deshalb scheint ein vollständiger verlust der S-Adenosylmethionin-Synthetase-Aktivität für die Zelle letal zu sein.

Durch eine Abschwächung des Gens metK oder der Aktivität der S-Adenosylmethionin-Synthetase kann eine Senkung des Abbaus von Methionin und damit eine Verringerung der Menge des gebildeten S-Adenosylmethionins sowie eine Verringerung der Inhibition der an der Methionin-Biosynthese beteiligten Produkte der Gene metB, metX und metY erreicht werden. Das resultiert in einer Steigerung der Methionin-Produktion durch die Zelle.

Vor kurzem wurde das Enzym S-Adenosylmethionin-Synthetase aus Corynebacterium glutamicum durch Mutation der dafür kodierenden DNA-Sequenz dahingehend verändert, dass das entstandene Protein eine deutlich verringerte Aktivität im Vergleich zum Wildtyp-Enzym aufweist (WO03/100072). Aus der Grafik in Figur 1 dieser Anmeldung ist für die veränderte S-Adenosylmethionin-Synthetase mit dem Aminosäureaustausch Cystein an Position 94 durch Alanin eine Aktivität von etwa 20% ersichtlich.

Die mikrobielle Biosynthese von L-Methionin und SAM in coryneformen Bakterien ist in Ablauf und Regulation äußerst komplex und darüber hinaus vielschichtig mit diversen anderen Stoffwechselwegen in der Zelle vernetzt. Daher kann im Voraus keine Vorhersage gemacht werden, mit welcher Restaktivität der S-Adenosylmethionin-Synthetase welcher Effekt auf das Wachstum eines Mikroorganismenstamms, die Balance seiner lebenswichtigen Stoffwechselabläufe und die Produktion von L-Methionin erzielt werden kann. Daher ist es wünschenswert, möglichst viele Varianten der S-Adenosylmethionin-Synthetase, die sich im Grad ihrer Aktivität unterscheiden, zur Verfügung zu haben.

### Aufgabe der Erfindung

Aufgabe der vorliegenden Erfindung ist es, ein breites Spektrum neuer Varianten der S-Adenosylmethionin-Synthetase (MetK-Protein) aus coryneformen Bakterien, insbesondere Corynebacterium glutamicum, zur Verfügung zu stellen, die eine im Vergleich zum Wildtyp verringerte Aktivität besitzen. Diese Varianten besitzen im Vergleich zu einem S-Adenosylmethionin-Synthetase Wildtyp-Enzym mindestens eine Mutation und sind Gegenstand der vorliegenden Erfindung.

Weiterhin ist es Aufgabe dieser Erfindung, neue Maßnahmen zur verbesserten fermentativen Herstellung von L-Aminosäuren, insbesondere L-Methionin, bereitzustellen.

### Beschreibung der Erfindung

Werden im folgenden L-Aminosäuren oder Aminosäuren erwähnt, sind damit eine oder mehrere der proteinogenen Aminosäuren einschließlich ihrer Salze, ausgewählt aus der Gruppe L-Asparaginsäure, L-Asparagin, L-Threonin, L-Serin, L-Glutaminsäure, L-Glutamin, L-Glycin, L-Alanin, L-Cystein, L-Valin, L-Methionin, L-Isoleucin, L-Leucin, L-Tyrosin, L-Phenylalanin, L-Histidin, L-Lysin, L-Tryptophan, L-Arginin und L-Prolin gemeint. Besonders bevorzugt ist L-Methionin.

Unter proteinogenen Aminosäuren versteht man die Aminosäuren, die in natürlichen Proteinen, das heißt in Proteinen von Mikroorganismen, Pflanzen, Tieren und Menschen vorkommen. Sie dienen als Struktureinheiten für Proteine, in denen sie über Peptidbindungen miteinander verknüpft sind.

Werden im folgenden L-Methionin oder Methionin erwähnt, sind damit auch die Salze wie z.B. Methionin-Hydrochlorid oder Methionin-Sulfat gemeint.

Verschiedene Varianten eines Gens, beispielsweise die erfindungsgemäßen Varianten des metK-Gens, kodierend für Enzyme mit Aktivität einer S-Adenosylmethionin-Synthetase, werden auch als "Allele" bezeichnet (Römpp Lexikon der Biotechnologie und Gentechnik, 2. Auflage 1999, Thieme Verlag Stuttgart, Deutschland). Verschiedene Allele eines Gens zeichnen sich durch Unterschiede in der Nukleotidsequenz aus.

Gegenstand der Erfindung ist eine aus coryneformen Bakterien, insbesondere Corynebacterium glutamicum stammende für die S-Adenosylmethionin-Synthetase kodierende, replizierbare Nukleotidsequenz (DNA), wobei die zugehörige Aminosäuresequenz in der SEQ ID No. 2 an Position 200 jede proteinogene Aminosäure ausgenommen L-valin enthält.

Gegenstand der Erfindung ist weiterhin eine aus coryneformen Bakterien insbesondere Corynebacterium glutamicum stammende für die S-Adenosylmethionin-Synthetase kodierende, replizierbare Nukleotidsequenz (DNA), wobei die zugehörige Aminosäuresequenz an Position 200 L-Aspartat enthält, dargestellt in der SEQ ID No. 4.

Gegenstand der Erfindung ist weiterhin eine aus coryneformen Bakterien insbesondere Corynebacterium glutamicum stammende für die S-Adenosylmethionin-Synthetase kodierende, replizierbare Nukleotidsequenz (DNA), deren Basensequenz an der Position 599 Adenin enthält, dargestellt in SEQ ID No. 3.

Gegenstand der Erfindung ist weiterhin eine aus coryneformen Bakterien, insbesondere Corynebacterium glutamicum stammende für die S-Adenosylmethionin-Synthetase kodierende, replizierbare Nukleotidsequenz (DNA), wobei die zugehörige Aminosäuresequenz in der SEQ ID No. 2 an Position 322 jede proteinogene Aminosäure ausgenommen L-Isoleucin enthält.

Gegenstand der Erfindung ist weiterhin eine aus coryneformen Bakterien insbesondere Corynebacterium glutamicum stammende für die S-Adenosylmethionin-Synthetase kodierende, replizierbare Nukleotidsequenz (DNA), wobei die zugehörige Aminosäuresequenz an Position 322 L-Leucin enthält, dargestellt in der SEQ ID No. 6.

Gegenstand der Erfindung ist weiterhin eine aus coryneformen Bakterien insbesondere Corynebacterium glutamicum stammende für die S-Adenosylmethionin-Synthetase kodierende, replizierbare Nukleotidsequenz (DNA), deren Basensequenz an der Position 964 Cytosin enthält, dargestellt in SEQ ID No. 5.

Gegenstand der Erfindung ist weiterhin eine aus coryneformen Bakterien insbesondere Corynebacterium glutamicum stammende für die S-Adenosylmethionin-Synthetase kodierende, replizierbare Nukleotidsequenz (DNA), wobei in der Basensequenz in der SEQ ID No. 2 an der Position 1201 die Base Cytosin deletiert ist. Aufgrund der daraus resultierenden Verschiebung des Leserasters der Nukleotidsequenz verlängert sich der in SEQ ID No. 2 dargestellte kodierende Bereich des metK-Gens um 13 Nukleotide der Abfolge "taaaaatctgatg", wobei "t" für Thymin steht, "a" für Adenin, "c" für Cytosin und "g" für Guanin. Die Sequenz ist in der SEQ ID No. 7 dargestellt.

Gegenstand der Erfindung ist weiterhin eine aus coryneformen Bakterien insbesondere Corynebacterium glutamicum stammende für die S-Adenosylmethionin-Synthetase kodierende, replizierbare Nukleotidsequenz (DNA), wobei die zugehörige Aminosäuresequenz um 4 Aminosäuren länger ist als die in SEQ ID No. 2 dargestellte und an den Positionen 401 bis 411 der Aminosäuresequenz die Aminosäuren L-Alanin, L-Glutamin, L-Prolin, L-Serin, L-Serin, L-Cystein, L-Prolin, L-Lysin, L-Asparagin, L-Leucin und L-Methionin trägt. Die Sequenz ist in SEQ ID No. 8 dargestellt.

Gegenstand der Erfindung sind weiterhin Plasmide (Vektoren), die die erfindungsgemäßen Nukleotidsequenzen enthalten und gegebenenfalls in coryneformen Bakterien replizieren.

Gegenstand der Erfindung sind weiterhin isolierte coryneforme Bakterien, die die erfindungsgemäßen Nukleotidsequenzen beziehungsweise die von diesen kodierten S-Adenosylmethionin Synthetase Enzymproteine mit abgeschwächten katalytischen Eigenschaften enthalten und/oder in denen die für die S-Adenosylmethionin-Synthetase kodierenden Nukleotidsequenzen abgeschwächt exprimiert werden.

Der Begriff "Abschwächung" bzw. "Abschwächen" beschreibt in diesem Zusammenhang die Verringerung oder Ausschaltung der intrazellulären Aktivität eines oder mehrerer Enzyme bzw. Proteine in einem Mikroorganismus, die durch die entsprechende DNA kodiert werden, indem man beispielsweise ein Gen bzw. Alle1 verwendet, das für ein entsprechendes Enzym mit einer niedrigen Aktivität kodiert bzw. das entsprechende Gen oder Enzym bzw. Protein inaktiviert und gegebenenfalls diese Maßnahmen kombiniert.

Durch die Maßnahmen der Abschwächung wird die Aktivität oder Konzentration des entsprechenden Proteins im allgemeinen auf 0 bis 75%, 0 bis 50%, 0 bis 25%, 0 bis 10% oder 0 bis 5% der Aktivität oder Konzentration des Wildtyp-Proteins, beziehungsweise der Aktivität oder Konzentration des Proteins im Ausgangs-Mikroorganismus, herabgesenkt.

Die Herabsetzung der Proteinkonzentration ist über 1- und 2-dimensionale Proteingelauftrennung und anschließende optische Identifizierung der Proteinkonzentration mit enstprechender Auswertesoftware im Gel nachweisbar. Eine gebräuchliche Methode zur Präparation der Proteingele bei coryneformen Bakterien und zur Identifizierung der Proteine ist die von Hermann et al. (Electrophoresis, 22:1712-23 (2001)) beschriebene Vorgehensweise. Die Proteinkonzentration kann ebenfalls durch Western-Blot-Hybridisierung mit einem für das nachzuweisende Protein spezifischen Antikörper (Sambrook et al., Molecular Cloning: A Laboratory Manual. 2^{nd} Ed., Cold Spring Harbor Laboratory Press, Cold Spring Harbor, N.Y., 1989) und anschließender optischer Auswertung mit entsprechender Software zur Konzentrationsbestimmung (Lohaus und Meyer (1998) Biospektrum 5:32-39; Lottspeich (1999) Angewandte Chemie 111:2630-2647) analysiert werden. Die Aktivität von DNA-bindenden Proteinen kann mittels DNA Band-Shift-Assays (auch als Gelretardation bezeichnet) gemessen werden wie beispielsweise im Lehrbuch "Bioanalytik" (Lottspeich/Zorbas, Spektrum Akademischer Verlag GmbH, Heidelberg, Deutschland (1998)) beschrieben und bei Wilson et al. (Journal of Bacteriology 183:2151-2155 (2001)) angewendet. Die Wirkung von DNA-bindenden Proteinen auf die Expression anderer Gene kann durch verschiedene gut beschriebene Methoden des Reportergen-Assays nachgewiesen werden (Sambrook et al., Molecular Cloning: A Laboratory Manual. 2^{nd} Ed., Cold Spring Harbor Laboratory Press, Cold Spring Harbor, N.Y., 1989).

Die Abschwächung der katalytischen Eigenschaft eines Proteins ist über verschiedene Enzymtests nachzuweisen. Für die Bestimmung der Aktivität der S-Adenosylmethionin-Synthetase können unterschiedliche Enzymtests benutzt werden. Bei der enzymatischen Umsetzung von Methionin zu SAM wird ATP zu Pyrophosphat und Orthophosphat umgesetzt. Ein geeigneter Test basiert auf dem photometrischen Nachweis von anorganischem Phosphat über einen Molybdatkomplex und ist bei Fiske und Subbarrow beschrieben (Journal of Biology and Chemistry 66: 375-400 (1925). Er basiert auf der Bildung eines blauen Molybdatkomplexes durch Molybdat und Orthophosphat (freies anorganisches Phosphat) unter reduzierenden Bedingungen.

Zur Erzielung einer Abschwächung können entweder die Expression des metK-Gens oder die katalytischen Eigenschaften der Genprodukte herabgesetzt oder ausgeschaltet werden. Gegebenenfalls werden beide Maßnahmen kombiniert.

Die Genexpression kann durch geeignete Kulturführung oder durch genetische Veränderung (Mutation) der Signalstrukturen der Genexpression verringert werden. Signalstrukturen der Genexpression sind beispielsweise Repressorgene, Aktivatorgene, Operatoren, Promotoren, Attenuatoren, Ribosomenbindungsstellen, das Startkodon und Terminatoren. Angaben hierzu findet der Fachmann z.B. in der Patentanmeldung WO 96/15246, bei Boyd und Murphy (Journal of Bacteriology 170: 5949-5952 (1988)), bei Voskuil und Chambliss (Nucleic Acids Research 26: 3584-3590 (1998), bei Pátek et al. (Microbiology 142: 1297-309 (1996) und Journal of Biotechnology 104: 311-323 (2003)) und in bekannten Lehrbüchern der Genetik und Molekularbiologie wie z.B. dem Lehrbuch von Knippers ("Molekulare Genetik", 6. Auflage, Georg Thieme Verlag, Stuttgart, Deutschland, 1995) oder dem von Winnacker ("Gene und Klone", VCH Verlagsgesellschaft, Weinheim, Deutschland, 1990).

Ein Beispiel für die gezielte Regulation der Genexpression ist die Klonierung des abzuschwächenden Gens unter die Kontrolle eines durch Zugabe dosierter Mengen von IPTG (Isopropyl-β-D-thiogalactopyranosid) induzierbaren Promotors wie zum Beispiel des trc-Promotors oder des tac-Promotors. Hierzu eignen sich Vektoren wie beispielsweise der Escherichia coli Expressionsvektor pXK99E (WO0226787; hinterlegt gemäß Budapester Vertrag am 31. Juli 2001 in DH5alpha/pXK99E als DSM14440 bei der Deutschen Sammlung für Mikroorganismen und Zellkulturen (DSMZ, Braunschweig, Deutschland)) oder pVWEx2 (Wendisch, Ph. D. thesis, Berichte des Forschungszentrums Jülich, Jül-3397, ISSN 0994-2952, Jülich, Deutschland (1997)), die eine IPTGabhängige Expression des klonierten Gens in Corynebacterium glutamicum ermöglichen.

Eingesetzt wurde diese Methode beispielsweise in der Patentanmeldung WO0226787 zur regulierten Expression des deaD-Gens durch Integration des Vektors pXK99EdeaD in das Genom von Corynebacterium glutamicum und von Simic et al. (Applied and Environmental Microbiology 68: 3321-3327 (2002)) zur regulierten Expression des glyA-Gens durch Integration des Vektors pK18mobglyA' in Corynebacterium glutamicum.

Eine weitere Methode zur spezifischen Verringerung der Genexpression ist die Antisense-Technik, wobei kurze Oligvdesoxyukleotide oder Vektoren zur Synthese längerer Antisense-RNA in die zielzellen gebracht werden. Die Antisense-RNA kann dort an komplementäre Abschnitte spezifischer mRNAs binden und deren Stabilität verringern oder die Translatierbarkeit blocken. Ein Beispiel hierzu findet der Fachmann bei Srivastava et al. (Applied Environmental Microbiology 2000 Oct; 66 (10): 4366 - 4371).

Um verschiedene Allele eines Gens, kodierend für Enzymproteine mit veränderten katalytischen Eigenschaften zu erhalten, können Mutationen auf unterschiedliche Weise in ein Gen integriert werden.

Beispielsweise können für die Mutagenese klassische in vivo Mutageneseverfahren unter Verwendung mutagener Stoffe wie beispielsweise N-Methyl-N'-Nitrv-N-Nitrosoguanidin (NTG), Ethyl-Methansulfonat (EMS) oder ultraviolettes Licht verwendet werden. Diese und weitere Methoden sind unter anderem im Manual of Methods for General Bacteriology (American Society for Microbiology, Washington, 1981) beschrieben.

Außerdem können für die Mutagenese in vitro Methoden wie beispielsweise eine Behandlung mit Hydroxylamin (Miller, J.H.: A Short Course in Bacterial Genetics. A Laboratory Manual and Handbook for Escherichia coli and related Bacteria, Cold Spring Harbor Laboratory Press, Cold Spring Harbor, 1992) oder die Polymerasekettenreaktion (PCR), wie sie im Handbuch von Newton und Graham (PCR, Spektrum Akademischer Verlag, Heidelberg, 1994) beschrieben ist, verwendet werden.

Eine weitere Möglichkeit zum Einführen zufälliger Mutationen in ein Gen ist der Einsatz spezifischer DNA-Polymerasen, die bei der Amplifikation eines Gens per PCR eine hohe Fehlerquote aufweisen (z.B. Mutazyme DNA Polymerase im GeneMorph PCR Mutagenesis Kit von Stratagene). Auf diese Weise kann ein breites Mutationsspektrum hergestellt werden. Die mutierten Gene können später mit gentechnischen Methoden wieder in den Ausgangsorganismus eingebracht werden.

Weitere Anleitungen zur Erzeugung von Mutationen können dem Stand der Technik und bekannten Lehrbüchern der Genetik und Molekularbiologie wie z.B. dem Lehrbuch von Knippers ("Molekulare Genetik", 6. Auflage, Georg Thieme Verlag, Stuttgart, Deutschland, 1995), dem von Winnacker ("Gene und Klone", VCH Verlagsgesellschaft, Weinheim, Deutschland, 1990) oder dem von Hagemann ("Allgemeine Genetik", Gustav Fischer Verlag, Stuttgart, 1986) entnommen werden.

Nach der Mutagenisierung können die Zellen auf Agarplatten aufgebracht werden und nach Inkubation einem Screening auf die gewünschten Eigenschaften unterzogen werden. So kann beispielsweise über Replika-Plattierung der Zellen auf entsprechenden selektiven Medien auf bestimmte Eigenschaften wie Resistenzen gegen bestimmte Antimetabolite, Auxotrophien für verschiedene Metabolite oder die Fähigkeit zur Verwertung bestimmter Zucker selektioniert werden. Ein Beispiel hierfür ist in Patent DE 4203320 beschrieben, in dem der Stamm Corynebacterium glutamicum DSM5714 mutagenisiert wird und durch Screening Mutanten mit erhöhter Aktivität des Sekretionssystems für L-Lysin gefunden werden.

Durch gezieltes Sequenzieren einzelner Gene können andererseits verschiedene Allele eines Gens ausfindig gemacht werden.

Zur Erzeugung der erfindungsgemäßen metK-Allele, die für Varianten der S-Adenosyl-Methionin-Synthetase, gekennzeichnet durch einen Aminosäureaustausch an Position 200 oder Position 322 oder eine auf einer Basen-Deletion an Position 1201 der Nukleotidsequenz beruhende Verlängerung der Aminosäuresequenz auf 411 Aminosäuren mit Austauschen ab Position 401 kodieren, werden im Stand der Technik beschriebene Mutagenesemethoden verwendet.

Um Enzyme mit abgeschwächter Aktivität der S-Adenosylmethionin-Synthetase zu erhalten, werden verschiedene Enzyme mit Aktivität der S-Adenosylmethionin-Synthetase, kodiert von Allelen des Gens metK, welche durch die beschriebenen Mutagenesemethoden gewonnen wurden, mit Hilfe des bei Fiske und Subbarrow (Journal of Biology and Chemistry 66: 375-400 (1925)) beschriebenen Tests zum Nachweis von anorganischem Phosphat auf ihre Aktivität überprüft. Allele des Gens metK, kodierend für Enzyme mit reduzierter Aktivität der S-Adenosylmethionin-Synthetase im Vergleich zum Wildtyp-Enzym, können mit gentechnischen Methoden wie z.B. der von Schwarzer und Pühler (Bio/Technology 9, 84-87 (1991)) beschriebene Methode des Genaustausches ("gene replacement") wieder in den Ausgangsorganismus oder andere Stämme der Gattung Corynebacterium eingebracht werden. Diese Methode ist ein gebräuchliches Verfahren, um Gene von C. glutamicum gezielt zu mutieren.

Mutationen können Transitionen, Transversionen, Insertionen und Deletionen sein. In Abhängigkeit von der Wirkung des Aminosäureaustausches auf die Enzymaktivität wird von Fehlsinnmutationen ("missense mutations") oder Nichtsinrunutationen ("nonsense mutations") gesprochen. Insertionen oder Deletionen von mindestens einem Basenpaar in einem Gen führen zu Rasterverschiebungsmutationen ("frame shift mutations"), in deren Folge falsche Aminosäuren eingebaut werden oder die Translation vorzeitig abbricht. Deletionen von mehreren Kodonen führen typischerweise zu einem vollständigen Ausfall der Enzymaktivität. Anleitungen zur Erzeugung derartiger Mutationen gehören zum Stand der Technik und können bekannten Lehrbüchern der Genetik und Molekularbiologie wie z.B. dem Lehrbuch von Knippers ("Molekulare Genetik", 6. Auflage, Georg Thieme Verlag, Stuttgart, Deutschland, 1995), dem von Winnacker ("Gene und Klone", VCH Verlagsgesellschaft, Weinheim, Deutschland, 1990) oder dem von Hagemann ("Allgemeine Genetik", Gustav Fischer Verlag, Stuttgart, 1986) entnommen werden.

Bei der Methode des Genaustausches ("gene replacement") wird eine Mutation wie z.B. eine Deletion, Insertion oder Basenaustausch in dem interessierenden Gen in-vitro hergestellt und hergestellte Allel in einen für C. glutamicum nicht replikativen Vektor kloniert. Als Vektoren kommen beispielsweise pK18mob, pK19mob, pK18mobsacB oder pK19mobsacB (Schäfer et al., Gene 145, 69-73 (1994)) oder pESB30 (Onishi et al., Applied Microbiology and Biotechnology 58: 217-223 (2002)) in Frage. Dieser vektor wird anschließend durch Konjugation oder Transformation in den gewünschten Wirt von C. glutamicum überführt. Die Methode der Konjugation ist beispielsweise bei Schäfer et al. (Journal of Bacteriology 172: 1663-1666 (1990) und Applied and Environmental Microbiology 60: 756-759 (1994)) beschrieben. Methoden zur Transformation sind beispielsweise bei Thierbach et al. (Applied Microbiology and Biotechnology 29, 356-362 (1988)), Dunican und Shivnan (Biotechnology 7, 1067-1070 (1989)) und Tauch et al. (FEMS Microbiological Letters 123, 343-347 (1994)) beschrieben. Nach homologer Rekombination mittels eines ersten, Integration bewirkenden "cross-over"-Ereignisses und eines geeigneten zweiten, eine Exzision bewirkenden "cross-over"-Ereignisses im zielgen bzw. in der zielsequenz erreicht man den Einbau der Mutation bzw. des Allels. Diese Methode ist bei Schwarzer und Pühler (Bio/Technology 9: 84-87 (1991) beschrieben und wurde beispielsweise von Möckel et al. verwendet, um unter anderem im ilvA-Gen von Corynebacterium glutamicum die Aminosäure valin an Position 323 der Aminosäuresequenz gegen Alanin auszutauschen (Molecular Microbiology Sep; 13(5): 833-842 (1994). Onishi et al. (Applied Microbiology and Biotechnology 58: 217-223 (2002)) führten unter Einsatz des Vektors pESB30 und mit Hilfe der Transformation in Corynebacterium glutamicum Punktmutationen in die Gene hom, lysC und pyc ein.

Einen Überblick über verschiedene gentechnische Verfahren wie Transformation, Konjugation, verschiedene Kloniervektoren und Selbstklonierung bei C. glutamicum geben Kirchner und Tauch (Journal of Biotechnology 104: 287-299 (2003).

In das metK-Gen kann auf diese Weise eine Deletion, Insertion oder ein Basenaustausch integriert werden.

Gegenstand der Erfindung sind neue, für Varianten der S-Adenosylmethionin-Synthetase kodierende replizierbare, bevorzugt endogene Nukleotidsequenzen (DNA), die in SEQ ID No. 3, SEQ ID No. 5 und SEQ ID No. 7 dargestellt sind.

Unter "endogenen Genen" oder "endogenen Nukleotidsequenzen" versteht man die in der Population einer Art vorhandenen Gene beziehungsweise Nukleotidsequenzen und deren Allele.

Die Erfindung betrifft ebenso Vektoren (Plasmide) die die genannten Nukleotidsquenzen enthalten und gegebenenfalls in coryneformen Bakterien replizieren.

Beansprucht werden auch coryneforme Bakterien, in denen die genannten Nukleotidsequenz(en) gemäß den für S-Adenosylmethionin-Synthetase kodierenden Nukleotidsequenzen bevorzugt überexprimiert vorliegen.

Gegenstand der Erfindung ist ein Verfahren zur Herstellung von L-Methionin oder L-Methionin enthaltenden Futtermitteladditiven, bei dem man folgende Schritte durchführt:
a) Fermentation coryneformer Bakterien, in denen Allele des endogenen metK-Gens überexprimiert werden unter Bedingungen, die geeignet sind, das metK-Genprodukt S-Adenosylmethionin-Synthetase zu bilden.
b) Isolieren des L-Methionins oder des L-Methionin enthaltenden Futtermitteladditivs aus der Fermentationsbrühe, wobei die coryneformen Bakterien das L-Methionin bilden, gegebenenfalls
c) mit Bestandteilen aus der Fermentationsbrühe und/oder der Biomasse (< bis 100%).

Ein weiterer Gegenstand dieser Erfindung ist ein Verfahren zur Herstellung von L-Methionin oder L-Methionin enthaltenden Futtermitteladditive, bei dem man im allgemeinen folgende Schritte durchführt:
a) Fermentation coryneformer Bakterien, die für die S-Adenosylmethionin-Synthetase kodierende endogene Nukleotidsequenzen enthalten, wobei in den zugehörigen Aminosäuresequenzen entweder das L-Valin an der Position 200 durch eine andere proteinogene Aminosäure, bevorzugt L-Aspartat, ersetzt ist, oder das L-Isoleucin an der Position 322 durch eine andere proteinogene Aminosäure, bevorzugt L-Leucin, ersetzt ist, oder das Protein um 4 Aminosäuren gegenüber dem Wildtyp-Protein verlängert ist und von Position 401 bis 411 der Aminosäure-Sequenz die Aminosäuren L-Alanin, L-Glutamin, L-Prolin, L-Serin, L-Serin, L-Cystein, L-Prolin, L-Lysin, L-Asparagin, L-Leucin und L-Methionin trägt.
   Die Allele des endogenen metK-Gens werden unter Bedingungen überexprimiert, die für die Bildung des metK-Genprodukts S-Adenosylmethionin-Synthetase geeignet sind.
b) Anreicherung des L-Methionins in der Fermentationsbrühe,
c) Isolierung des L-Methionins oder L-Methionin enthaltenden Futtermitteladditivs aus der Fermentationsbrühe, gegebenenfalls
d) mit Bestandteilen aus der Fermentationsbrühe und/oder der Biomasse (> 0 bis 100 %).

Unter proteinogenen Aminosäuren sind sämtliche Aminosäuren zu verstehen, die Bestandteile von Proteinen beziehungsweise Polypeptiden sind. Insbesondere sind dies: L-Asparaginsäure, L-Asparagin, L-Threonin, L-Serin, L-Glutaminsäure, L-Glutamin, Glycin, L-Alanin, L-Cystein, L-Valin, L-Methionin, L-Isoleucin, L-Leucin, L-Tyrosin, L-Phenylalanin, L-Histidin, L-Lysin, L-Tryptophan, L-Prolin und L-Arginin.

Die Wildform des metK-Gens ist in Wildtypstämmen coryneformer Bakterien insbesondere der Gattung Corynebacterium enthalten. Sie ist in der SEQ ID No. 1 dargestellt. Das Wildtyp-Protein ist in der SEQ ID No. 2 dargestellt.

Bei der Gattung Corynebacterium ist insbesondere die in der Fachwelt bekannte Art Corynebacterium glutamicum zu nennen. Bekannte Wildtypstämme der Art Corynebacterium glutamicum sind beispielsweise
Corynebacterium glutamicum ATCC13032
Corynebacterium acetoglutamicum ATCC15806 Corynebacterium acetoacidophilum ATCC13870
Corynebacterium melassecola ATCC17965
Corynebacterium thermoaminogenes FERM BP-1539
Brevibacterium flavum ATCC14067
Brevibacterium lactofermentum ATCC13869 und
Brevibacterium divaricatum ATCC14020,
oder wie beispielsweise der L-Methionin produzierende Stamm
Corynebacterium glutamicum ATCC21608.

Stämme mit der Bezeichnung "ATCC" können von der American Type Culture Collection (Manassas, VA, USA) bezogen werden. Stämme mit der Bezeichnung "FERM" können vom National Institute of Advanced Industrial Science and Technology (AIST Tsukuba Central 6, 1-1-1 Higashi, Tsukuba Ibaraki, Japan) bezogen werden. Der genannte Stamm von Corynebacterium thermoaminogenes (FERM BP-1539) und weitere (FERM BP-1540, FERM BP-1541 und FERM BP-1542) sind in der US-A 5,250,434 beschrieben.

Zur Erzeugung der erfindungsgemäßen metK-Allele, die für Varianten der S-Adenosyl-Methionin-Synthetase, gekennzeichnet durch einen Aminosäureaustausch an Position 200 oder Position 322 oder eine auf einer Basen-Deletion an Position 1201 der Nukleotidsequenz beruhende Verlängerung der Aminosäuresequenz auf 411 Aminosäuren mit Austauschen ab Position 401 kodieren, werden im Stand der Technik beschriebene Mutagenesemethoden verwendet.

Bei Verwendung von in-vitro Methoden wird das im Stand der Technik beschriebene metK-Gen ausgehend von isolierter Gesamt-DNA eines Wildtypstammes mit Hilfe der Polymerasekettenreaktion amplifiziert, gegebenenfalls in geeignete Plasmidvektoren kloniert, und die DNA anschließend dem Mutageneseverfahren unterworfen. Anleitungen zur Amplifikation von DNA-Sequenzen mit Hilfe der Polymerase-Kettenreaktion (PCR) findet der Fachmann unter anderem im Handbuch von Gait: Oligonucleotide Synthesis: A Practical Approach (IRL Press, Oxford, UK, 1984) und bei Newton und Graham: PCR (Spektrum Akademischer Verlag, Heidelberg, Deutschland, 1994). Geeignete metK-Allele werden anschließend mit den oben beschriebenen Verfahren ausgelesen und untersucht.

Gegenstand der Erfindung sind neue, für Varianten der S-Adenosylmethionin-Synthetase kodierende metK-Allele, die in SEQ ID No. 3, SEQ ID No. 5 und SEQ ID No. 7 dargestellt sind.

Zusätzlich kann es für die Produktion von L-Aminosäuren vorteilhaft sein, zusätzlich zur Verwendung der erfindungsgemäßen metK-Allele gleichzeitig eines oder mehrere Enzyme des jeweiligen Biosyntheseweges, der Glykolyse, der Anaplerotik, des Zitronensäure-Zyklus, des Pentosephosphat-Zyklus, des Aminosaure-Exports und gegebenenfalls regulatorische Proteine entweder zu verstärken, insbesondere überzuexprimieren, oder abzuschwächen, insbesondere auszuschalten oder die Expression zu verringern. Dabei können auch verschiedene Allele dieser Gene eingesetzt werden, welche beispielsweise für Proteine (Enzyme) mit verstärkter oder abgeschwächter Aktivität im Vergleich zum Wildtyp-Protein kodieren oder welche für Proteine (Enzyme) mit verringerter Hemmbarkeit durch verschiedene Produkte im Vergleich zum Wildtyp-Protein kodieren und welche sich vorteilhaft auf die Produktion von Aminosäuren, insbesondere L-Methionin, auswirken. Die Verwendung endogener Gene wird im allgemeinen bevorzugt,

Unter "endogenen Genen" oder "endogenen Nukleotidsequenzen" versteht man die in der Population einer Art vorhandenen Gene beziehungsweise Nukleotidsequenzen und deren Allele.

Der Begriff "Verstärkung" beschreibt in diesem Zusammenhang die Erhöhung der intrazellulären Aktivität oder Konzentration eines oder mehrerer Enzyme beziehungsweise Proteine in einem Mikroorganismus, die durch die entsprechende DNA kodiert werden, indem man beispielsweise die Kopienzahl des Gens bzw. der Gene erhöht, einen starken Promotor verwendet oder ein Gen oder Allel verwendet, das für ein entsprechendes Enzym beziehungsweise Protein mit einer hohen Aktivität kodiert und gegebenenfalls diese Maßnahmen kombiniert. Eine Erhöhung der Aktivität des entsprechenden Enzymproteins kann auch durch eine verringerte Sensibilität gegenüber Inhibitoren bewirkt werden.

Durch die Maßnahmen der Verstärkung, insbesondere Überexpression, wird die Aktivität oder Konzentration des entsprechenden Proteins im allgemeinen um mindestens 10%, 25%, 50%, 75%, 100%, 150%, 200%, 300%, 400% oder 500%, maximal bis 1000% oder 2000% bezogen auf die des wildtyp-Proteins beziehungsweise der Aktivität oder Konzentration des Proteins im Ausgangs-Mikroorganismus erhöht.

So kann für die Herstellung von L-Methionin zusätzlich zur Verwendung einer Variante des metK-Gens gleichzeitig eines oder mehrere der Gene, ausgewählt aus der Gruppe der Gene der Methioninproduktion verstärkt, insbesondere überexprimiert werden. Unter "Gene oder Allele der Methioninproduktion" sind sämtliche, bevorzugt endogene, offene Leserahmen, Gene oder Allele zu verstehen, deren Verstärkung/Überexpression eine Verbesserung der Methioninproduktion bewirken kann.

Hierzu gehören unter anderem folgende offene Leserahmen, Gene oder Allele: accBC, accDA, aecD, cstA, cysD, cysE, cysH, cysK, cysN, cysQ, dps, eno, fda, gap, gap2, gdh, gnd, glyA, hom, hom^{FBR}, lysC, lysC^{FBR}, metA, metB, metE, metH, metY, msiK, opcA, oxyR, ppc, ppc^{FBR}, pgk, pknA, pknB, pknD, pknG, ppsA, ptsH, ptsI, ptsM, pyc, pyc P458S, sigC, sigD, sigE, sigH, sigM, tal, thyA, tkt, tpi, zwa1, zwf und zwf A213T. Diese sind in Tabelle 1 zusammengefasst und erläutert.

**Tabelle 1 Gene und Allele der Methioninproduktion**

| Name | Bezeichnung des kodierten Enzyms oder Proteins | Referenz | Zugangs nummer |
|---|---|---|---|
| accBC | Acyl-CoA Carboxylase EC 6.3.4.14 (acyl-CoA carboxylase) | Jäger et al. Archives of Microbiology (1996) 166:76-82 EP1108790; WO0100805 | U35023 AX123524 AX066441 |
| accDA | Acetyl-CoA Carboxylase EC 6.4.1.2 (acetyl-CoA carboxylase) | EP1055725 EP1108790 WO0100805 | AX121013 AX066443 |
| aecD | Cystathionin beta-Lyase EC 4.4.1.8 (cystathionine beta-lyase) | Rossol et al., Journal of Bacteriology 174:2968-2977 (1992) | M89931 |
| cstA | Carbon Starvation Protein A (carbon starvation protein A) | EP1108790 WO0100804 | AX120811 AX066109 |
| cysD | Sulfat-Adenylyltransferase Untereinheit II EC 2.7.7.4 (sulfate adenylyltransferase small chain) | EP1108790 | AX123177 |
| cysE | Serinacetyltransferase EC 2.3.1.30 (serine acetyltransferase) | EP1108790 WO0100843 | AX122902 AX063961 |
| cysH | 3'-Phosphoadenylsulfat Reduktase EC 1.8.99.4 (3'-phosphoadenosine 5'- phosphosulfate reductase) | EP1108790 WO0100842 | AX123178 AX066001 |
| cysK | Cystein-Synthase EC 4.2.99.8 (cysteine synthase) | EP1108790 WO0100843 | AX122901 AX063963 |
| cysN | Sulfat-Adenylyltransferase Untereinheit I EC 2.7.7.4 (sulfate adenylyltransferase) | EP1108790 | AX123176 AX127152 |
| cysQ | Transportprotein CysQ (transporter cysQ) | EP1108790 WO0100805 | AX127145 AX066423 |
| dps | DNA-Protection Protein (protection during starvation protein) | EP1108790 | AX127153 |
| Eno | Enolase EC 4.2.1.11 (enolase) | EP1108790 WO0100844 EP1090998 Hermann et al., Electrophoresis 19:3217-3221 (1998) | AX127146 AX064945 AX136862 |
| Fda | Fruktose Bisphosghat Aldolase EC 4.1.2.13 (fructose bisphosphate aldolase) | van der Osten et al., Molecular Microbiology 3:1625-1637 (1989) | X17313 |
| gap | Glyceraldehyd-3-Phosphat Dehydrogenase EC 1.2.1.12 (glyceraldehyde-3-phosphate dehydrogenase) | EP1108790 WO0100844 Eikmanns et al., Journal of Bacteriology 174:6076-6086(1992) | AX127148 AX064941 X59403 |
| gap2 | Glyceraldehyd-3-Phosphate Dehydrogenase EC 1.2.1.12 (glyceraldehydc-3-phosphate dehydrogenase 2) | EP1108790 WO0100844 | AX127146 AX064939 |
| gdh | Glutamat Dehydrogenase EC 1.4.1.4 (glutamate dehydrogenase) | EP1108790 WO0100844 Boermann et al., Molecular Microbiology 6:317-326 (1992) | AX127150 AX063811 X59404 X72855 |
| glyA | Glycine/Serin Hydroxymethyltransferase EC 2.1.2.1 (glycine/serine hydroxymethyltransferase) | EP1108790 | AX127146 AX121194 |
| gnd | 6-Phosphogluconat Dehydrogenase EC 1.1.1.44 (6-phosphogluconate dehydrogenase) | EP1108790 WO0100844 | AX127147 AX121689 AX065125 |
| hom | Homoserin Dehydrogenase EC 1.1.1.3 (homoserine dehydrogenase) | Peoples et al., Molecular Microbiology 2:63-72 (1988) | Y00546 |
| hom^{FBR} | Homoserin Dehydrogenase feedback resistent (fbr) EC 1.1.1.3 (homoserine dehydrogenase fbr) | Reinscheid et al., Journal of Bacteriology 173:3228-30 (1991) | |
| lysC | Aspartatkinase EC 2.7.2.4 (aspaztate kinase) | EP1108790 WO0100844 Kalinowski et al., Molecular Microbiology 5:1197-204 (1991) | AX120365 AX063743 X57226 |
| LysC^{FBR} | Aspartatkinase feedback resistent (fbr) EC 2.7-2.4 (aspartate kinase fbr) | Siehe Tabelle 2 | |
| metA | Homoserin Acetyltransferase EC 2.3.1.31 (homoserine acctyltransferase) | Park et al., Molecular Cells 8:286-94 (1998) | AF052652 |
| MetB | Cystahionin γ-Lyase EC 4.4.1.1 (cystathionine gamma-synthase) | Hwang et al., Molecular Cells 9:300-308 (1999) | AF126953 |
| metE | Homocystein-Methyltransferase EC 2.1.1.14 (homacysteine methyltransferase) | EP1108790 | AX127146 AX121345 |
| metH | Homocystein-Methyltransferase (Vitamin B12 abhängig) EC 2.1.1.14 (homocysteine methyltransferase) | EP1108790 | AX127148 AX121747 |
| metY | Acetylhomoserin Sulfhydrolase (acetylhomoserine sulfhydrolase) | EP1108790 | AX120810 AX127145 |
| msiK | Zuckerimporter (multiple sugar import protein) | EP1108790 | AX120892 |
| opcA | Glukose-6-Phosphat-Dehydrogenase (subunit of glucose-6-phosphate dehydrogenase) | WO0104325 | AX076272 |
| oxyR | Transcriptionsregulator (transcriptional regulator) | EP1108790 | AX122198 AX127149 |
| ppc^{FBR} | Phosphoenolpyruvat Carboxylase feedback resistent EC 4.1.1.31 (phosphoenolpyruvate carboxylase feedback resistant) | EP0723011 WO0100852 | |
| ppc | Phosphoenolpyruvat Carboxylase EC 4.1.1.31 (phosphoenolpyruvate carboxylase) | EP1108790 O'Reagan et al., Gene 77(2):237-251(1989) | AX127148 AX123554 M25819 |
| pgk | Phosphoglycerat Kinase EC 2.7.2.3 (phosphoglycerate kinase) | EP1108790 WO0100844 Eikmanns, Journal of Bacteriology 174:6076-6086 (1992) | AX121838 AX127148 AX064943 X59403 |
| pknA | Protein kinase A (protein kinase A) | EP1108790 | AX120131 AX120085 |
| pknB | Protein kinase B (protein kinase B) | EP1108790 | AX120130 AX120085 |
| pknD | Protein kinase D (protein kinase D) | EP1108790 | AX127150 AX122469 AX122468 |
| pknG | Protein kinase G (protein kinase G) | EP1108790 | AX127152 AX123109 |
| ppsA | Phosphoenolpyruvat-Synthase EC 2.7.9.2 (phosphoenolpyruvate synthase) | EP1108790 | AX127144 AX120700 AX122469 |
| ptsH | Phosphotransferase System Protein H EC 2.7.1.69 (phosphotransferase system component H) | EP1108790 WO0100844 | AX122210 AX127149 AX069154 |
| ptsI | Phosphotransferase System Enzym I EC 2.7.3.9 (phosphotransferase system enzyme I) | EP1108790 | AX122206 AX127149 |
| ptsM | Glukose spezifisches Phosphotransferase System Enzym II EC 2.7.1.69 (glucose-phosphotransferase-system enzyme II) | Lee et al., FEMS Microbiology Letters 119(1-2):137-145 (1994) | L18874 |
| pyc | Pyruvat-Carboxylase EC 6.4.1.1 (pyruvate carboxylase) | WO9918228 Peters-Wenclisch et al., Microbiology 144:915-927 (1998) | A97276 Y09548 |
| pyc P458S | Pyruvat-Carboxylase EC 6.4.1.1 (pyruvate carboxylase) Aminosäureaustausch P458S | EP1108790 | |
| sigC | Sigmafaktor C EC 2.7.7.6 (extracytoplasmic function alternative sigma factor C) | EP1108790 | AX120368 AX120085 |
| sigD | RNA Polymerase Sigmafaktor D EC 2.7.7.6 (RNA polymerase sigma factor) | EP1108790 | AX120753 AX127144 |
| sigE | Sigmafaktor E EC 2.7.7.6 (extracytoplasmic function alternative sigma factor E) | EP1108790 | AX127146 AX121325 |
| sigH | Sigmafaktor H EC 2.7.7.6 (sigma factor SigH) | EP1108790 | AX127145 AX120939 |
| sigM | Sigmafaktor M EC 2.7.7.6 (sigma factor SigM) | EP1108790 | AX123500 AX127153 |
| tal | Transaldolase EC 2.2.1.2 (transaldolase) | WO0104325 | AX076272 |
| thyA | Thymidylat Synthasc EC 2.1.1.45 (thymidylate synthase) | EP1108790 | AX121026 AX127145 |
| tkt | Transketolase EC 2.2.1.1 (transketolase) | Ikeda et al., NCBI | AB023377 |
| tpi | Triose-phosphat Isomerase EC 5.3.1.1 (triose-phosphate isomerase) | Eikmanns, Journal of Bacteriology 174:5076-6086 (1992) | X59403 |
| zwa1 | Zellwachstumsfaktor 1 (growth factor 1) | EP1111062 | AX133781 |
| zwf | Glukose-6-phosphat-1-Dehydrogenasc EC 1.1.1.49 (glucose-6-phosphate-1- dehydrogenase) | EP1108790 WO0104325 | AX127148 AX121827 AX076272 |
| zwf A213T | Glukose-6-phosphat-1-Dehydrogenase EC 1.1.1.49 (glucose-6-phosphate-1- dehydrogenase) Aminosäureaustausch A213T | EP1108790 | |

Weiterhin kann es für die Produktion von L-Methionin vorteilhaft sein, neben der Verwendung einer Variante des metK-Gens gleichzeitig eines oder mehrere der Gene, ausgewählt aus der Gruppe der Gene oder Allele, die für das Wachstum oder die Methioninproduktion nicht essentiell sind, abzuschwächen, insbesondere auszuschalten oder die Expression zu verringern.

Hierzu gehören unter anderem folgende offene Leserahmen, Gene oder Allele: ccpA1, ccpA2, citA, citB, citE, ddh, gluA, gluB, gluC, gluD, luxR, luxS, lysR1, lysR2, lysR3, menE, metD, metK, pck, pgi, poxB und zwa2. Diese sind in Tabelle 2 zusammengefasst und erläutert.

**Tabelle 2 Gene und Allele, die für die Methioninproduktion nicht essentiell sind**

| Gen- name | Bezeichnung des kodierten Enzyms oder Proteins | Referenz | Zugangs nummer |
|---|---|---|---|
| ccpA1 | Katabolic-Koneroll-Protein (catabolite control protein Al) | WO0100844 EP1108790 | AX065267 AX127147 |
| ccpA2 | Katabolit-Kontroll-Protein (catabolite control protein A2) | WO0100844 EP1108790 | AX065267 AX121594 |
| citA | Sensor-Kinase CitA (sensor kinase CitA) | EP1108790 | AX120161 |
| citB | Transkriptionsregulator CitB (transcription regulator CitB) | EP1108790 | AX120163 |
| citE | Citrat-Lyase EC 4.1.3.6 (citrate lyase) | WO0100844 EP1108790 | AX065421 AX127146 |
| ddh | Diaminopimelat-Dehydrogenase EC 1.4.1.16 (diaminopimelate dehydrogenase) | Ishino et al., Nucleic Acids Research 15: 3917 (1987) EP1108790 | S07384 AX127152 |
| gluA | Glutamat-Transport ATP-bindendes Protein (glutamate transport ATP-binding protein) | Kronemeyer et al., Journal of Bacteriology 177(5):1152-8 (1995) | X81191 |
| gluB | Glutamait-bindendes Protein (glutamate binding protein) | Kronemeyer et al., Journal of Bacteriology 177(5):1152-8 (1995) | X81191 |
| gluC | Glutamat-Transport Permease (glutamate transport system permease) | Kronemeyer et al., Journal of Bacteriology 177(5):1152-8 (1995) | X81191 |
| gluD | Glutamat-Transport Permease (glutamate transport system permease) | Kronemeyer et al., Journal of Bacteriology 177(5):1152-8 (1995) | X81191 |
| luxR | Transkriptionsregulator LuxR (transcription regulator LuxR) | WO0100842 EP1108790 | AX065953 AX123320 |
| luxS | Histidin-Kinase LuxS (hirtidinc kinase LuxS) | EP1108790 | AX123323 AX127153 |
| lysR1 | Transkriptionsregulator LysR1 (transcription regulator LysR1) | EP1108790 | AX064673 AX127144 |
| lysR2 | Transkriptionsaktivator LysR2 (transcription regulator LysR2) | EP1108790 | AX123312 |
| lysR3 | Transkriptionsregulator LysR3 (transcription regulator LysR3) | WO0100842 EP1108790 | AX065957 AX127150 |
| menE | O-Succinylbenzoesäure-CoA-Ligase EC 6.2.1.26 (O-succinylbenzoate-CoA ligase) | W00100843 EP1108790 | AX064599 AX064193 AX127144 |
| metD | Transkriptionsregulator MetD (transcription regulator MetD) | EP1108790 | AX123327 AX127153 |
| metK | Methionin-Adenosyl- Transferase EC 2.5.1.6 (S-adenosylmethioninc synthetase) | WO0100843 EP1108790 | AX063959 AX127148 |
| pck | Phosphoenolpyruvat- Carboxykinase (phosphoenolpyruvate carboxykinase) | WO0100844 | AJ269506 AX065053 |
| pgi | Glucose-6-Phosphat- Isomerase EC 5.3.1.9 (glucose-6-phosphate isomerase) | EP1087015 EP1108790 | AX136015 AX127146 |
| poxB | Pyruvat-Oxidase EC 1.2.3.3 (pyruvate oxidase) | WO0100844 EP1096013 | AX064959 AX137665 |
| zwa2 | Zellwachstumsfaktor 2 (growth factor 2) | EP1106693 EP1108790 | Ax113822 AX127146 |

Schließlich kann es für die Produktion von Aminosäuren, insbesondere L-Methionin vorteilhaft sein, neben der Verwendung einer Variante des metK-Gens unerwünschte Nebenreaktionen auszuschalten (Nakayama: "Breeding of Amino Acid Producing Microorganisms", in: Overproduction of Microbial Products, Krumphanzl, Sikyta, Vanek (eds.), Academic Press, London, UK, 1982).

Die erfindungsgemäß hergestellten Mikroorganismen sind ebenfalls Gegenstand der Erfindung und können kontinuierlich oder diskontinuierlich im batch - verfahren (Satzkultivierung) oder im fed batch (Zulaufverfahren) oder repeated fed batch Verfahren (repetitives Zulaufverfahren) zum Zwecke der Produktion von L-Aminosäuren kultiviert werden. Eine Zusammenfassung über bekannte Kultivierungsmethoden ist im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) beschrieben.

Das zu verwendende Kulturmedium muss in geeigneter weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten.

Als Kohlenstoffquelle können Zucker und Kohlehydrate wie z.B. Glucose, Saccharose, Laktose, Fructose, Maltose, Melasse, Stärke und Cellulose, öle und Fette, wie zum Beispiel Sojaöl, Sonnenblumenöl, Erdnußöl und Kokosfett, Fettsäuren, wie zum Beispiel Palmitinsäure, Stearinsäure und Linolsäure, Alkohole wie zum Beispiel Glycerin und Ethanol und organische Säuren, wie zum Beispiel Essigsäure verwendet werden. Diese Stoffe können einzeln oder als Mischung verwendet werden.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium haltigen Salze verwendet werden. Das Kulturmedium muss weiterhin Salze von Metallen enthalten, wie zum Beispiel Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter weise während der Kultivierung zugefüttert werden.

Zur pH - Kontrolle der Kultur werden basische Verbindungen wie Natriumhydroxid, Kaliumhydroxid, Ammoniak beziehungsweise Ammoniakwasser oder saure Verbindungen wie Phosphorsäure oder Schwefelsäure in geeigneter Weise eingesetzt. Zur Kontrolle der Schaumentwicklung können Antischaummittel, wie zum Beispiel Fettsäurepolyglykolester eingesetzt werden. Zur Aufrechterhaltung der Stabilität von Plasmiden können dem Medium geeignete selektiv wirkende Stoffe, wie zum Beispiel Antibiotika hinzugefügt werden. Um aerobe Bedingungen aufrechtzuerhalten, werden Sauerstoff oder Sauerstoff-haltige Gasmischungen, wie zum Beispiel Luft in die Kultur eingetragen. Die Temperatur der Kultur liegt normalerweise bei 20°C bis 45°C und vorzugsweise bei 25°C bis 40°C. Die Kultur wird solange fortgesetzt, bis sich ein Maximum des gewünschten Produktes gebildet hat. Dieses Ziel wird normalerweise innerhalb von 10 Stunden bis 160 Stunden erreicht.

Methoden zur Bestimmung von L-Aminosäuren sind aus dem Stand der Technik bekannt. Die Analyse kann zum Beispiel so wie bei Spackman et al. (Analytical Chemistry, 30, (1958), 1190) beschrieben durch Anionenaustausch-Chromatographie mit anschließender Ninhydrin-Derivatisierung erfolgen, oder sie kann durch reversed phase HPLC erfolgen, so wie bei Lindroth et al. (Analytical Chemistry (1979) 51: 1167-1174) beschrieben. Informationen dazu findet der Fachmann auch bei Ashman et al. (in: Tschesche (Hrsg), Modern Methods in Protein Chemistry. 155-172, de Gruyter, Berlin 1985).

Das erfindungsgemäße Verfahren dient zur fermentativen Herstellung von L-Methionin.

Die Konzentration von L-Methionin kann gegebenenfalls durch den Zusatz von L-Methionin auf den gewünschten Wert eingestellt werden.

Die vorliegende Erfindung wird im folgenden anhand von Ausführungsbeispielen näher erläutert.

### Beispiel 1

### Einschleusung einer Deletion in das Gen ccpA1 vom Stamm ATCC13032ΔmetD

### 1.1 Konstruktion des Austauschvektors pK18mobsacBΔccpA1

Bei dem für die Versuche verwendeten Ausgangsstamm handelt es sich um den Stamm ATCC13032AmetD, der eine Deletion in dem für den Transkriptionsregulator MetD codierenden Gen trägt. Seine Konstruktion ist in WO02097096 beschrieben.

Die Sequenz dieses Gens ccpA1 ist bekannt und aus der Patentanmeldung US683267 ersichtlich.

Zur Einschleusung einer Deletion in das Gen ccpA1 wurde chromosomale DNA aus dem Stamm ATCC13032 nach dem Verfahren von Eikmanns et al. (Microbiology 140: 1817-1828 (1994)) isoliert. Ausgehend von der Sequenz des Gens ccpA1 wurden die unten beschriebenen Oligonukleotide zur Erzeugung des ccpA1-Deletionsallels mittels Polymerase-Kettenreaktion (PCR) mit der Gen-Soeing-Methode (Horton, Molecular Biotechnology 3: 93-98 (1995)) gewählt.

Primer ccpA1-del1 (siehe auch SEQ ID No. 9):
5´-GCTA-GAATTC-AAGGCCACATTGACCAGTTC -3´

Primer ccpA1-del2 (siehe auch SEQ ID No. 10):
5′-TGTGACCTAGGCTTGACTTT-ATTTAAAT-ATGATCCTGCACCAATCCTT -3′

Primer ccpA1-del3 (siehe auch SEQ ID No. 11):
5´-AAGGATTGGTGCAGGATCAT-ATTTAAAT-AAAGTCAAGCCTAGGTCACA -3´

Primer ccpA1-del4 (siehe auch SEQ ID No. 12):
5´-GCTA-GAATTC-ATCTCATCCAAGTGGTGCGA -3´

Die angegebenen Primer wurden von MWG Biotech (Ebersberg, Deutschland) synthetisiert und die PCR-Reaktion wurde nach der Standard-PCR-Methode von Innis et al. (PCR Protocols. A Guide to Methods and Applications, 1990, Academic Press) mit Platinum Pfx DNA Polymerase (Invitrogen, Karlsruhe, Deutschland) durchgeführt.

Jeder der beiden Primer ccpA1-del1 und ccpA1-del4 enthält in jedem Fall eine eingefügte Spaltstelle für das Restriktionsenzym EcoRI, und die Primer ccpA1-del2 und ccpA1-del3 eine eingefügte Spaltstelle für das Restriktionsenzym SwaI, die in der oben gezeigten Nukleotidsequenz unterstrichen sind.

Der Primer ccpA1-del2 besteht aus zwei Regionen der Nukleotidsequenz, von denen eine an die "upstream"-Region vor dem Start-Codon von ccpA1 bindet und die andere an die "dowmstream"-Region nach dem Stop-Codon von ccpA1 bindet. Die beiden Regionen sind durch die eingefügte SwaI-Restriktionsstelle getrennt. Der Primer ccpA1-del3 ist zu dem Primer ccpA1-del2 revers komplementär.

Mit Hilfe der Polymerasekettenreaktion ermöglichen die Primer ccpA1-del1 und ccpA1-del2 die Amplifikation eines 513 Basenpaare (Bp) langen DNA-Fragments und die Primer ccpA1-del3 und ccpA1-del4 ermöglichen die Amplifikation eines 651 Bp langen DNA-Fragments. Die Amplifikate wurden durch anschließende Agarosegel-Elektrophorese in 0,8%igem Agarosegel überprüft, aus dem Agarosegel mit dem QIAquick Gel Extraction Kit (Qiagen, Hilden, Deutschland) isoliert und gemeinsam als DNA-Matrize in einer weiteren PCR-Reaktion mit den Primern ccpA1-del1 und ccpA1-del4 verwendet. Dies ergab ein 1116 Bp großes ccpA1-Deletionsderivat. Das amplifizierte Produkt wurde anschließend in einem 0,8%igem Agarosegel überprüft.

Das ccpA1-Deletionsderivat wurde mit der Restriktionsendonuklease EcoRI gespalten, nach Überprüfung in einem 0,8%igem Agarosegel mit dem QIAquick Gel Extraction Kit (Qiagen, Hilden, Deutschland) aus dem Agarosegel isoliert und für die Ligation mit dem von Schäfer et al., Gene, 14, 69-73 (1994) beschriebenen mobilisierbaren Klonierungsvektor pK18mobsacB verwendet. Der Vektor pK18mobsacB wurde zuvor mit dem Restriktionsenzym EcoRI gespalten und anschließend mit alkalischer Phosphatase aus Shrimp (Roche Diagnostics GmbH, Mannheim, Deutschland, Product Description SAP, Produkt Nr. 1758250) entphosphoryliert. Der vorbereitete Vektor wurde anschließend mit dem ccpAl-Deletionsderivat vermischt und mit T4 DNA-Ligase (Amersham-Pharmacia, Freiburg, Deutschland) behandelt.

Der E. coli-Stamm DH5αmcr (Grant, 1990, Proceedings of the National Academy of Sciences U.S.A., 87: 4645-4649) wurde anschließend gemeinsam mit dem Ligationsansatz elektroporiert (Hanahan, In. DNA cloning. A practical approach. Band 1. ILR-Press, Cold Spring Harbor, New York, 1989). zellen, die das Plasmid trugen, wurden dadurch selektiert, daß man den Transformationsansatz LB-Agar (Merck, Darmstadt, Deutschland), der mit 25 mg/l Kanamycin versetzt worden war, ausplattierte.

Von einer Tranzformante wurde Plasmid-DNA mit Hilfe des QIAprep Spin Miniprep Kit von Qiagen isoliert und durch Schneiden mit den Restriktionsenzymen EcoRI und SwaI überprüft. Das Plasmid wird pK18mobsacBΔccpA1 genannt. Der Stamm wird E.coli DH5αmcr/pK18mobsacB ΔccpA1 genannt.

### 1.2. Deletionsmutagenese des ccpAl-Gens im C. Glutamicum-Stamm ATCC13032ΔmetD

Der in Beispiel 1.1 genannte Vektor pK18mobsacBΔccpA1 wurde nach der Elektroporationsmethode von Tauch et al., (1989 FEMS Microbiology Letters 123: 343-347) in den Stamm C. glutamicum ATCC13032ΔmetD elektroporiert. Der Vektor kann nicht selbständig in ATCC13032ΔmetD replizieren und verbleibt nur dann in der Zelle, wenn er in das Chromosom integriert hat. Klone mit integriertem pK18mobsacB ΔccpA1 werden dadurch selektiert, daß man den Elektroporationsansatz auf LB-Agar (Merck, Darmstadt, Deutschland), das mit 15 mg/l Kanamycin versetzt worden war, ausplattierte. Weitergewachsene Klone wurden auf LB-Agarplatten mit 25 mg/l Kanamycin ausplattiert und 16 Stunden bei 33°C inkubiert.

Um ein Herausschneiden des Plasmids zusammen mit der vollständigen chromosomalen Kopie des ccpA1-Gens zu erzielen, wurden die Klone unselektiv über Nacht in LB-Medium (Merck, Darmstadt, Deutschland) inkubiert und dann auf LB-Agar mit 10% Saccharose gezüchtet. Das Plasmid pK18mobsacB enthält eine Kopie des sacB-Gens, das Saccharose in Levansaccharose, die für C. glutamicum toxisch ist, umwandelt. Nur diejenigen Klone, in denen das integrierte pK18mobsacBΔccpA1 wieder herausgeschnitten worden ist, wachsen daher auf LB-Agar mit Saccharose. Bei dem Herausschneiden kann gemeinsam mit dem Plasmid entweder die vollständige chromosomale Kopie des ccpA1-Gens oder das ccpAl-Deletionsderivat herausgeschnitten werden.

Um zu zeigen, daß das ccpA1-Gen im Chromosom deletiert worden ist, wurde nach dem Herausschneiden des Austauschvektors die ccpA1-Region von 10 unterschiedlichen Klonen mittels PCR amplifiziert. Zuerst wurde chromosomale DNA aus den 10 Klonen nach der Methode von Eikmanns et al. (Microbiology 140: 1817-1828 (1994)) isoliert. Für die PCR-Reaktion wurden die folgenden Primer verwendet:

ccpA1-A1 (siehe auch SEQ ID No. 13):

5´-CCTGCACACCACCGCTGAAT-3´

ccpA1-E1 (siehe auch SEQ ID No. 14):

5´-CAGCCGCACCAGCTCATCTT-3´

Die angegebenen Primer wurden von MWG Biotech (Ebersberg, Deutschland) synthetisiert und die PCR-Reaktion wurde nach der Standard-PCR-Methode von Innis et al. (PCR Protocols. A Guide to Methods and Applications, 1990, Academic Press) mit Platinum Pfx DNA Polymerase (Invitrogen, Karlsruhe, Deutschland) durchgeführt.

Der Primer ccpA1-A1 bindet an die "upstream"-Region von ccpA1 vor dem ccpA1-Deletionsderivat und der Primer ccpA1-E1 bindet an die "downstream"-Region nach dem ccpA1-Deletionsderivat. Ist die Deletion des ccpA1-Gens in das Genom von Stamm ATCC13032ΔmetD eingeschleust, so ist das amplifizierte PCR-Fragment ungefähr 1,4 kB groß. Liegt das ccpA1-Wildtypgen vor, so ist das amplifizierte PCR-Fragment ungefähr 2,7 kB groß.

Auf diese weise wurde mit dem beschriebenen PCR-Verfahren ein Klon mit dem amplifizierten, ungefähr 1,4 kB großen ccpAl-Deletionsfragment identifiziert. Dieser Klon wird C. glutamicum ATCC13032ΔmetDΔccpA1 genannt.

### Beipiel 2

### Einschleusung der Mutation metKI322L in den Stamm ATCC13032ΔmetDΔccpA1

Die in SEQ. ID. No. 5 gezeigte Nukleotidsequenz des metK-Allels enthält eine Adenin-Cytosin-Transversion in Position 964 der Nukleotidsequenz, was zu einem Aminosäurenaustausch von Isoleucin gegen Leucin an Position 322 der erhaltenen Aminosäurensequenz führt. Diese Mutation wird im folgenden als metKI322L bezeichnet.

### 2.1 Konstruktion des Austauschvektors pK18mobsacB_metKI322L

Zuerst wurde aus dem Stamm ATCC13032 nach dem Verfahren von Eikmanns et al. (Microbiology 140: 1817-1828 (1994)) chromosomale DNA isoliert. Ausgehend von der Sequenz des Gens metK und der umgebenden Regionen, die für Corynebacterium glutamicum bekannt sind, wurden die unten beschriebenen Oligonukleotide für die Herstellung des metKI322L-Austauschallels gewählt.

metK-1 (siehe auch SEQ. ID. No. 15):

5'- TGCGACCTCCTCATGTGTAA-3'

metK-2 (siehe auch SEQ. ID. No. 16):

5´-AGGTTGTCGTCGCCATCATT-3´

Die angegebenen Primer wurden von MWG Biotech (Ebersberg, Deutschland) synthetisiert und die PCR-Reaktion wurde nach der Standard-PCR-Methode von Innis et al. (PCR Protocols. A Guide to Methods and Applications, 1990, Academic Press) mit Platinum Pfx DNA Polymerase (Invitrogen, Karlsruhe, Deutschland) durchgeführt.

Der Primer metK-1 bindet an die "upstream"-Region vor dem Start-Codon von metK und der Primer metK-2 bindet an die "downstream"-Region nach dem Stop-Codon von metK.

Mit Hilfe der Polymerasekettenreaktion ermöglichten die Primer metK-1 und metK-2 die Amplifikation eines ungefähr 2,3 kB großen DNA-Fragments, das das metK-Gen enthielt.

Das Amplifikat wurde durch anschließende Agarosegelelektrophorese in einem 0,8% Agarosegel überprüft. Dann wurde das ungefähr 2,3 kB große Amplifikat mit dem Zero Blunt TOPO Kit (Invitrogen, Carlsbad, CA, USA) in den Vektor pCR®Blunt II-TOPO ligiert. Anschließend wurde der E. coli-Stamm Top10 (Grant et al., Proceedings of the National Academy of Sciences USA, 87 (1990) 4645-4649) unter Verwendung des Handbuchs des Herstellers mit dem Ligationsansatz transformiert. Plasmidtragende Zellen wurden dadurch selektiert, daß man den Transformationsansatz auf LB-Agar, der mit 25 mg/l Kanamycin versetzt worden war, ausplattierte (Sambrock et *al*., Molecular Cloning, a laboratory manual. 2. Ausgabe, Cold Spring Harbor, New York, 1989). Plasmid-DNA wurde mit dem Eppendorf Perfectprep Plasmid Mini Kit (Eppendorf, Hamburg, Deutschland) isoliert und mittels Restriktionsspaltung mit der Restriktionsendonuklease EcoRI überprüft.

Die Nukleotidsequenz des klonierten DNA-Fragments, die das metK-Gen enthält, wurde von Agowa (Berlin, Deutschland) bestimmt. Das erhaltene Plasmid wurde pCRII_metK genannt.

Mit dem QuikChange-Kit für die ortsgerichtete Mutagenese (Stratagene, La Jolla, Ca, USA) wurde unter Verwendung des Handbuchs des Herstellers eine ortsgerichtete Mutagenese durchgeführt, um das Isoleucin an Position 322 der Aminosäuresequenz des metK-Gens gegen Leucin auszutauschen. Mit Hilfe der Nukleotidsequenz des metK-Wildtypgens und des Allels metKI322L (siehe SEQ. ID. No. 1 und SEQ. ID. No. 5) wurden die folgenden Primer für die lineare Amplifikation gestaltet:

metK-I322La (siehe auch SEQ ID No. 17):

5'-CAGGTTGCATACGCC(CTT)GGACGCGCAAAG-3'

metK-I322Lb (siehe auch SEQ ID No. 18):

5'-CTTTGCGCGTCC(AAG)GGCGTATGCAACCTG-3'

Die angegebenen Primer wurden von MWG Biotech (Ebersberg, Deutschland) synthetisiert. Das Codon für Leucin, das Isoleucin an Position 322 ersetzen soll, ist in der oben gezeigten Nukleotidsequenz durch Klammern angegeben. Das Plasmid pCRII_metK, das in diesem Beispiel beschrieben wird, wurde gemeinsam mit den beiden Primern, die jeweils zu einem Strang des Plasmids komplementär sind, für die lineare Amplifikation mittels PfuTurbo DNA Polymerase verwendet. Durch Primer-Verlängerung erhielt man ein mutiertes Plasmid mit Nicks in den ringförmigen Strängen. Das Produkt der linearen Amplifikation wurde mit DpnI behandelt; diese Endonuklease schneidet spezifisch die methylierte und halbmethylierte Matrizen-DNA. Die neu synthetisierte, Nicks aufweisende, mutierte Vektor-DNA wurde in den E. coli-Stamm XL1 Blue (Bullock, Fernandez und Short, BioTechniques (5): 376-379 (1987)) transformiert. Nach der Transformation wurde von den XL1-Blue-Zellen eine Reparatur der Nicks in dem mutierten Plasmid durchgeführt. Die Transformanten wurden auf LB-Medium (Merck, Darmstadt, Deutschland), das 100 mg/l Kanamycin enthielt, selektiert. Nach der Isolation von Plasmid-DNA von einer Transformante wurde das Plasmid mit der Restriktionsendonuklease EcoRI gespalten und durch anschließende Agarosegelelektrophorese in einem 0,8%igen Agarosegel überprüft.

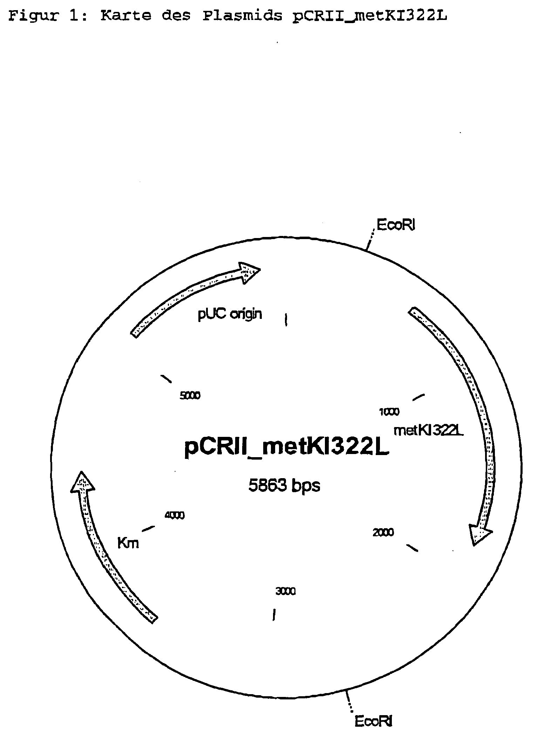

Die Nukleotidsequenz des metK-DNA-Fragments auf dem isolierten Plasmid, die das metK-Allel, das die metKI322L-Mutation trug, enthielt, im folgenden als "metKI322L-Allel" bezeichnet, wurde von Agowa (Berlin, Deutschland) bestimmt. Das erhaltene Plasmid wird pCRII_metKI322L genannt und ist in Abbildung 1 dargestellt.

Das klonierte DNA-Fragment, das das metKI322L-Allel trägt, wurde durch vollständige Spaltung des Vektors pCRII_metKI322L mit dem Restriktionsenzym EcoRI isoliert. Nach Auftrennung in einem Agarosegel (0.8%ig) wurde das ungefähr 2,4 kB große DNA-Fragment, das das metKI322L-Allel trug, mit Hilfe des QIAquick Gel Extraction Kit (Qiagen, Hilden, Deutschland) aus dem Agarosegel isoliert.

Das so behandelte DNA-Fragment, das das metKI322L-Allel trug, wurde für die Ligation mit mobilisierbarem Klonierungsvektor pK18mobsacB (Schäfer et al., Gen 14: 69-73 (1994)) verwendet. Dieser wurde zuvor mit dem Restriktionsenzym EcoRI gespalten und anschließend mit alkalischer Phosphatase aus Shrimp (Roche Diagnostics GmbH, Mannheim, Deutschland) entphosphoryliert. Die Vektor-DNA wurde mit dem DNA-Fragment vermischt und der Ansatz wurde mit T4-DNA-Ligase (Amersham- Pharmacia, Freiburg, Deutschland) behandelt.

Anschließend wurde der E. coli-Stamm DH5αmcr (Grant, 1990, Proceedings of the National Academy of Sciences U.S.A., 87: 4645-4649) mit dem Ligationsansatz transformiert. Plasmidtragende Zellen wurden dadurch selektiert, daß man den Transformationsansatz auf LB-Agar, der mit 50 mg/l Kanamycin versetzt worden war, ausplattierte (Sambrook et al., Molecular Cloning: A Laboratory Manual. 2. Ausgabe, Cold Spring Harbor, New York, 1989).

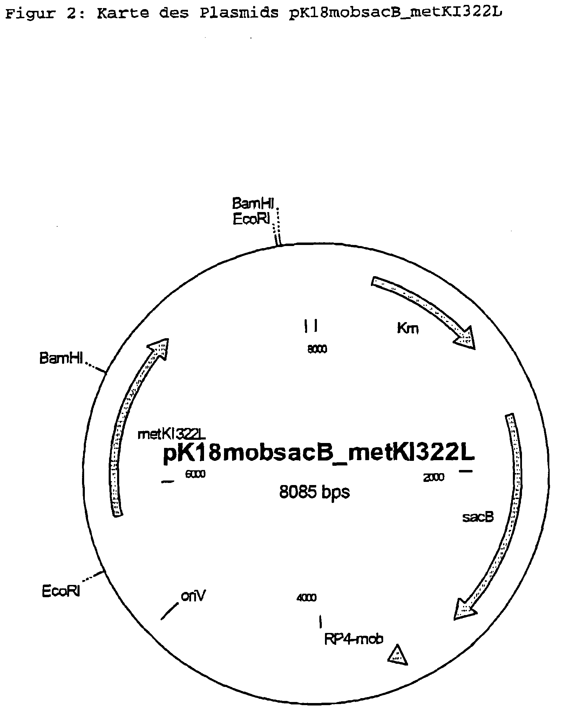

Von einer Transformante wurde Plasmid-DNA mit Hilfe des Eppendorf Perfectprep Plasmid Mini Kit (Eppendorf, Hamburg, Deutschland) isoliert und mittels Restriktionsspaltung, und zwar unter einmaliger Verwendung der Restriktionsendonuklease EcoRI und einmaliger Verwendung der Restriktionsendonuklease BamHI, überprüft. Das Plasmid wird pK18mobsacB_metKI322L genannt und ist in Abbildung 2 dargestellt. Der Stamm wird E. coli DH5αmcr/pK18mobsacB_metKI322L genannt.

### 2.2. Mutagenese des metK-Gens in dem C. glutamicum-Stamm ATCC13032ΔmetDΔccpA1

Der Vektor pK18mobsacB_metKI322L wurde nach der Elektroporationsmethode von Tauch et al., (1989 FEMS Microbiology Letters 123: 343-347) in den Stamm C. glutamicum ATCC13032ΔmetΔccpA1 elektroporiert. Der Vektor kann in ATCC13032ΔmetDΔccpA1 nicht selbständig replizieren und verbleibt nur dann in der Zelle, wenn er in das Chromosom integriert hat.

Transkonjuganten, d.h. Klone, die pK18mobsacB_metKI322L integriert haben, wurden dadurch selektiert, daß man den Konjugationsansatz auf LB-Agar, das mit 25 mg/l Kanamycin und 50 mg/l Nalidixinsäure ergänzt worden war, ausplattierte. Kanamycinresistente Transkonjugaten wurden dann auf LB-Agarplatten, die mit Kanamycin (25 mg/l) versetzt worden waren, ausgestrichen und 24 Stunden bei 33°C inkubiert. Für die Selektion von Mutanten, in denen aufgrund eines zweiten Rekombinationsereignisses ein Herausschneiden des Plasmids stattgefunden hatte, wurden die Klone 30 Stunden nicht selektiv in LB-Flüssigmedium gezüchtet und dann auf LB-Agar, der mit 10% Saccharose versetzt worden war, ausgestrichen und 24 Stunden bei 33°C inkubiert.

Wie das Elternplasmid pK18mobsacB enthält auch das Plasmid pK18mobsacB_metKI322L zusätzlich zu dem Kanamycinresistenzgen eine Kopie des sacB-Gens, das für die Levansaccharase aus Bacillus subtilis codiert. Die Expression des sacB-Gens, die durch Saccharose induzierbar ist, führt zur Bildung der Levansaccharase, die die Synthese des Produkts Levan, das für C. glutamicum toxisch ist, katalysiert. Es wachsen daher auf mit Saccharose versetztem LB-Agar nur diejenigen Klone, in denen das integrierte pK18mobsacB_metKI322L aufgrund eines zweiten Rekombinationsereignisses herausgeschnitten worden ist. Je nach der Lage des zweiten Rekombinationsereignisses in bezug auf die Mutationsstelle findet während des Herausschneidens entweder der Allelaustausch oder der Einbau der Mutation statt, oder das Wildtypallel verbleibt in dem wirtschromosom.

Anschließend wurde nach einem Klon gesucht, in dem der gewünschte Austausch, d.h. der Einbau der Mutation mit metKI322L, stattgefunden hatte. Zu diesem Zweck wurde an 10 Klonen mit dem Phänotyp "Wachstum in Gegenwart von Saccharose" und "kein wachstum in Gegenwart von Kanamycin" die Sequenz des metK-Gens bestimmt. Auf diese Weise wurde ein Klon, der die Mutation metKI322L trug, identifiziert. Dieser Stamm wurde C. glutamicum ATCC13032ΔmetDΔccpA1_metKI322L genannt.

### Beispiel 3

### Transformation der Stämme ATCC13032ΔmetDΔccpA1 und ATCC13032ΔmetDΔccpA1_meeKI322L mit dem Expressionsvektor pCREmetAEY

Der Expressionsvektor pCPEmetAEY (US6942996), der die Gene metX, metE und metY der Methioninbiosynthese von Corynebacterium glutamicum enthält, wurde nach der Elektroporationsmethode von Tauch et al. (1989 FEMS Microbiology Letters 123: 343-347) in die Stämme C. glutamicum ATCC13032ΔmetDΔccpA1_metKI322L und ATCC13032ΔmetDΔccpA1 elektroporiert. Plasmidtragende Zellen wurden dadurch selektiert, daß man den Transformationsansatz auf LB-Agar, der mit 25 mg/l Kanamycin versetzt worden war, ausplattierte (Sambrock et *al*., Molecular Cloning, a laboratory manual. 2. Ausgabe, Cold Spring Harbor, New York, 1989). Plasmid-DNA wurde nach traditionellen Verfahren (Peters-Wendisch et al., Microbiology 144: 915-927 (1998)) jeweils von einer kanamycinresistenten Transformante isoliert und mittels Restriktionsspaltung überprüft. Die erhaltenen Stämme wurden ATCC13032ΔmetDΔccpA1/pCREmetAEY und ATCC13032ΔmetDΔccpA1_metKI322L/pCREmetAEY genannt.

### Beispiel 4

### Produktion von Methionin

Die in Beispiel 3 erhaltenen C. glutamicum-Stämme ATCC13032ΔmetDΔccpA1/pCREmetAEY und ATCC13032ΔmetDΔccpA1_metKI322L/pCREmetAEY wurden in einem für die Produktion von Methionin geeigneten Nährmedium gezüchtet und der Methioningehalt im Kulturüberstand wurde bestimmt.

Zu diesem Zweck wurden die Stämme zuerst 24 Stunden bei 33°C auf einer LB-Agarplatte inkubiert. Ausgehend von dieser Agarplattenkultur wurde eine vorkultur inokuliert (10 ml Medium in einen 100-ml-Erlenmeyerkolben). Als Vorkulturmedium wird das MM-Medium verwendet.

**MM-Medium**

| | |
|---|---|
| Maisquellwasser (CSL, cornsteep liquor) | 5 g/l |
| MOPS (Morpholinopropansulfonsäure) | 20 g/l |
| Glucose (getrennt autoklaviert) | 50g/l |
| Salze: | |
| (NH₄)₂SO₄ | 25 g/l |
| KH₂PO₄ | 1,0 g/l |
| MgSO₄ * 7 H₂O | 1,0 g/l |
| CaCl₂ * 2 H₂O | 10 mg/l |
| FeSO₄ * 7 H₂O | 10 mg/l |
| MnSO₄ * H₂O | 5,0mg/l |
| Biotin (sterilfiltriert) | 0,01 mg/l |
| Vitamin B12 (sterilfiltriert) | 0,02 mg/l |
| Thiamin * HCl (sterilfiltriert) | 0,2 mg/l |
| CaCO₃ | 25 g/l |

Das Maisquellwasser, die MOPS und die Salzlösung wurden mit wäßrigem Ammoniak auf pH 7 gebracht und autoklaviert. Dann wurden die sterilen Substrat- und Vitaminlösungen sowie das im trockenen Zustand autoklavierte CaCO₃ zugegeben.

Die vorkultur wurde 16 Stunden bei 33°C und 240 U/min. auf einem Schüttler inkubiert. Mit dieser Vorkultur wurde eine Hauptkultur so inokuliert, daß die ursprüngliche OD (660 nm) der Hauptkultur 0,1 betrug. Für die Hauptkultur wurde ebenfalls MM-Medium verwendet.

Die Kultur wurde in einem Volumen von 10 ml in einem mit Schikanen versehenen 100-ml-Erlenmeyerkolben durchgeführt. Es wurde bei 33°C und 80% Luftfeuchtigkeit gearbeitet.

Nach 72 Stunden wurde die OD bei einer Meßwellenlänge von 660 nm mit einer Biomek 1000 (Beckmann Instruments GmbH, München) bestimmt. Die gebildete Methioninmenge wurde mit einem Aminosäureanalysator von Bppendorf-BioTronik (Hamburg, Deutschland) mittels Ionenaustauschchromatographie und Nachsäulenderivatisierung mit Ninhydrin-Detektion bestimmt.

Das Ergebnis des Versuchs ist in Tabelle 1 dargestellt.

**Tabelle 1**

| Stamm | OD (660 nm) | Methionin mg/l |
|---|---|---|
| ATCC13032ΔmetDΔccpA1/p CREmetAEY | 12,6 | 300 |
| ATCC13032ΔmetDΔccpA1_me tKI322L/pCREmetAEY | 12,8 | 510 |

Kurze Beschreibung der Abbildungen:
- Abbildung 1:: Karte des Plasmids pCRII_metKI322L
- Abbildung 2:: Karte des Plasmids pK18mobsacB_metKI322L

Die verwendeten Abkürzungen und Bezeichnungen haben die folgenden Bedeutungen. Ist die Anzahl Basenpaare (Bp) angegeben, so handelt es sich hierbei um einen ungefähren wert, der innerhalb der Reproduzierbarkeit der Messungen erhalten wurde.
- pUC origin: Replikationsursprung von pUC
- Km: Kanamycinresistenzgen
- metKI322L: kloniertes DNA-Fragment, das das metKI322L- Allel enthält
- sacB: sacB-Gen
- oriV: ColE1-Replikationsursprung von pMB1
- EcoRI: Spaltstelle des Restriktionsenzyms EcoRI
- BamHI: Spaltstelle des Restriktionsenzyms BamHI
- RP4-mob: RP4-Mobilisierungsstelle

## Patentansprüche

1. Aus coryneformen Bakterien stammende für ein Polypeptid mit S-Adenosylmethionin-Synthetase Enzymaktivität kodierende, replizierbare Nukleotidsequenzen (DNA), wobei in den zugehörigen Aminosäuresequenzen das L-Valin an Position 200 von SEQ ID No. 2 oder das L-Isoleucin an Position 322 von SEQ ID No. 2 durch eine andere proteinogene Aminosäure ersetzt wird.

2. Aus coryneformen Bakterien stammende für das Protein S-Adenosylmethionin-Synthetase kodierende, replizierbare Nukleotidsequenz (DNA) gemäß Anspruch 1, wobei die zugehörige Aminosäuresequenz an Position 200 L-Aspartat enthält (SEQ ID No. 4) oder an Position 322 L-Leucin enthält (SEQ ID No. 6) oder wobei das Protein um 4 Aminosäuren gegenüber dem wildtyp-Protein verlängert ist und von Position 401 bis 411 der Aminosäure-Sequenz die Aminosäuren L-Alanin, L-Glutamin, L-Prolin, L-Serin, L-Serin, L-Cystein, L-Prolin, L-Lysin, L-Asparagin, L-Leucin und L-Methionin trägt (SEQ ID No. 8).

3. Aus coryneformen Bakterien stammende für das Protein S-Adenosylmethionin-Synthetase kodierende, replizierbare Nukleotidsequenz (DNA) gemäß Anspruch 1, deren Basensequenz an der Position 599 Adenin enthält (SEQ ID No. 3) oder an der Position 964 Cytosin enthält (SEQ ID No. 5) oder das Cytosin an Position 1201 deletiert ist (SEQ ID No. 7).

4. Plasmide (Vektoren), die die Nukleotidsequenzen gemäß den Ansprüchen 1 bis 3 enthalten und gegebenenfalls in coryneformen Bakterien replizieren.

5. Coryneforme Bakterien, die Nukleotidsequenzen gemäß den Ansprüchen 1 bis 4 enthalten.

6. Bakterien, die den Vektor gemäß Anspruch 4 enthalten.

7. Isolierte coryneforme Bakterien, die für ein Polypeptid mit S-Adenosylmethionin-Synthetase Enzymaktivität kodierende Nukleotidsequenzen enthalten, wobei in den zugehörigen Aminosäuresequenzen das L-Valin an Position 200 von SEQ ID No. 2 oder das L-Isoleucin an Position 322 von SEQ ID No. 2 durch eine andere proteinogene Aminosäure ersetzt wird oder wobei das Protein um 4 Aminosäuren gegenüber dem Wildtyp-Protein verlängert ist und von Position 401 bis 411 der Aminosäure-Sequenz die Aminosäuren L-Alanin, L-Glutamin, L-Prolin, L-Serin, L-Serin, L-Cystein, L-Prolin, L-Lysin, L-Asparagin, L-Leucin und L-Methionin trägt.

8. Verfahren zur Herstellung von L-Methionin oder L-Methionin enthaltenden Futtermitteladditiven, bei dem man folgende Schritte durchführt:
a) Fermentation coryneformer Bakterien, in denen Allele des endogenen metK-Gens überexprimiert werden unter Bedingungen, die geeignet sind, das metK-Genprodukt S-Adenosylmethionin-Synthetase zu bilden;
b) Isolieren des L-Methionins oder des L-Methionin enthaltenden Futtermitteladditivs aus der Fermentationsbrühe, wobei die coryneformen Bakterien das L-Methionin bilden, gegebenenfalls
c) mit Bestandteilen aus der Fermentationsbrühe und/oder der Biomasse (< bis 100%).

9. verfahren gemäß Anspruch 8, **dadurch gekennzeichnet, dass** man coryneforme Bakterien einsetzt, die ein Allel des metK-Gens enthalten, wobei in den zugehörigen Aminosäuresequenzen das L-Valin an Position 200 von SEQ ID No. 2 oder das L-Isoleucin an Position 322 von SEQ ID No. 2 durch eine andere proteinogene Aminosäure ersetzt wird.

10. Verfahren gemäß Anspruch 8, **dadurch gekennzeichnet, dass** man Mikroorganismen einsetzt, in denen man zusätzlich weitere Gene des Biosyntheseweges des L-Methionins überexprimiert.

11. Verfahren gemäß Anspruch 8, **dadurch gekennzeichnet, dass** man zur Herstellung der L-Aminosäure oder des L-Aminosäure enthaltenden Futtermitteladditivs zusätzlich weitere Gene des Biosynthesewegs der L-Aminosäure überexprimiert.

12. Verfahren gemäß Anspruch 8, **dadurch gekennzeichnet, dass** man Mikroorganismen einsetzt, in denen die Stoffwechselwege zumindest teilweise abgeschwächt oder ausgeschaltet sind, die die Bildung des L-Methionins verringern.

13. Verfahren zur Herstellung von L-Methionin oder L-Methionin enthaltenden Futtermitteladditiven bei dem man folgende Schritte durchführt:
a) Fermentation coryneformer Bakterien, die für die S-Adenosylmethionin-Synthetase kodierende endogene Nukleotidsequenzen enthalten, wobei in den zugehörigen Aminosäuresequenzen das L-Valin an der Position 200 durch eine andere proteinogene Aminosäure ersetzt ist, bevorzugt L-Aspartat, oder das L-Isoleucin an der Position 322 durch eine andere proteinogene Aminosäure ersetzt ist, bevorzugt L-Leucin.
b) Anreicherung des L-Methionins in der Fermentationsbrühe,
c) Isolieren des L-Methionins oder des L-Methionin enthaltenden Futtermitteladditivs aus der Fermentationsbrühe, gegebenenfalls
d) mit Bestandteilen aus der Fermentationsbrühe und/oder der Biomasse (> 0 bis 100%).
